# EUROPEAN PATENT SPECIFICATION

(11) **EP 3 357 513 B1**
(45) Date of publication and mention of the grant of the patent: **25.10.2023**
(21) Application number: 16850363.9
(22) Date of filing: 28.09.2016
(51) Int. Cl.: A61K 45/06, A61P 35/04, A61K 31/454, A61K 31/4166, A61K 31/4035

(54) **PHARMACEUTICAL COMPOSITION AND APPLICATION THEREOF**
PHARMAZEUTISCHE ZUSAMMENSETZUNG UND VERABREICHUNG DAVON
COMPOSITION PHARMACEUTIQUE ET APPLICATION DE CETTE DERNIÈRE

(30) Priority: 29.09.2015 CN 201510631654
(43) Date of publication of application: 08.08.2018
(73) Proprietor: Kangpu Biopharmaceuticals, Ltd., Shanghai 201203 (CN)
(72) Inventor: GE, Chuansheng, Shanghai 201203 (CN); LIAO, Baisong, Shanghai 201203 (CN); LEE, Wen-Cherng, Shanghai 201203 (CN)
(74) Representative: dompatent von Kreisler Selting Werner - Partnerschaft von Patent- und Rechtsanwälten mbB
(86) International application number: PCT/CN2016/100642
(87) International publication number: WO 2017/054739

(56) References cited:
- WO-A1-02/059106
- WO-A1-2011/050962
- WO-A1-2014/152833
- US-A1- 2003 045 552
- US-A1- 2004 087 546
- US-A1- 2011 196 150
- US-A1- 2014 011 774
- US-A1- 2015 080 419
- William D. Figg ET AL: "A Randomized Phase II Trial of Thalidomide, an Angiogenesis Inhibitor, in Patients with Androgen-independent Prostate Cancer", Clinical Cancer Research, 1 July 2001 (2001-07-01), page 1888, XP055488059, United States Retrieved from the Internet: URL:http://clincancerres.aacrjournals.org/ content/clincanres/7/7/1888.full.pdf
- FIGG W D ET AL: "A Double-Blind Randomized Crossover Study of Oral Thalidomide Versus Placebo for Androgen Dependent Prostate Cancer Treated With Intermittent Androgen Ablation", JOURNAL OF UROLOGY, LIPPINCOTT WILLIAMS & WILKINS, BALTIMORE, MD, US, vol. 181, no. 3, 1 March 2009 (2009-03-01) , pages 1104-1113, XP025993684, ISSN: 0022-5347, DOI: 10.1016/J.JURO.2008.11.026 [retrieved on 2009-01-23]
- GULLEY J L ET AL: "Kinetics of Serum Androgen Normalization and Factors Associated With Testosterone Reserve After Limited Androgen Deprivation Therapy for Nonmetastatic Prostate Cancer", JOURNAL OF UROLOGY, LIPPINCOTT WILLIAMS & WILKINS, BALTIMORE, MD, US, vol. 180, no. 4, 1 October 2008 (2008-10-01), pages 1432-1437, XP025410332, ISSN: 0022-5347, DOI: 10.1016/J.JURO.2008.06.017 [retrieved on 2008-08-16]
- CHADI NABHAN ET AL: "Lenalidomide Monotherapy in Chemotherapy-Naive, Castration-Resistant Prostate Cancer Patients: Final Results of a Phase II Study", CLINICAL GENITOURINARY CANCER, vol. 12, no. 1, 1 February 2014 (2014-02-01), pages 27-32, XP055488660, US ISSN: 1558-7673, DOI: 10.1016/j.clgc.2013.09.001
- Jeanny B Aragon-Ching ET AL: "Thalidomide analogues as anticancer drugs", Recent patents on anti-cancer drug discovery, 1 June 2007 (2007-06-01), pages 167-174, XP055474095, United Arab Emirates Retrieved from the Internet: URL:https://www.ncbi.nlm.nih.gov/pmc/artic les/PMC2048745/
- CHADI NABHAN ET AL: "The Role of IMiDs Alone or in Combination in Prostate Cancer", CLINICAL GENITOURINARY CANCER, vol. 10, no. 3, 1 September 2012 (2012-09-01), pages 141-146, XP055488662, US ISSN: 1558-7673, DOI: 10.1016/j.clgc.2012.03.005
- ANDRESSA ARDIANI ET AL: "Androgen deprivation therapy sensitizes prostate cancer cells to T-cell killing through androgen receptor dependent modulation of the apoptotic pathway", ONCOTARGET, vol. 5, no. 19, 15 October 2014 (2014-10-15), XP055488676, United States ISSN: 1949-2553, DOI: 10.18632/oncotarget.2429
- MICHAEL E. JUNG ET AL: "Structure-Activity Relationship for Thiohydantoin Androgen Receptor Antagonists for Castration-Resistant Prostate Cancer (CRPC)", JOURNAL OF MEDICINAL CHEMISTRY, vol. 53, no. 7, 8 April 2010 (2010-04-08), pages 2779-2796, XP055122595, ISSN: 0022-2623, DOI: 10.1021/jm901488g
- FANG, LUO ET AL.: 'The New Progress of Global Antineoplastic Drug R&D' PROCEEDINGS OF ANTICANCER DRUG SYMPOSIUM ON RESEARCH PROGRESS AND PERSONALIZED THERAPY 31 December 2013, pages 211 - 233, XP009508158
- PANG, RAN ET AL.: 'The Progress of Treatment of HRPC' CHINESE JOURNAL OF SURGERY OF INTEGRATED TRADITIONAL AND WESTERN MEDICINE vol. 19, no. 1, 28 February 2013, pages 100 - 102, XP009505371

## Description

The present application claims the benefit of Chinese Patent Application No. CN201510631654.9 filed on September 29, 2015.

### Field of invention

The present invention relates to a pharmaceutical composition.

### Prior arts

Prostate cancer is a common malignancy in the male reproductive system. The statistics which was made by the International Agency for Research on Cancer of World Health Organization in 2012 showed that the number of newly diagnosed prostate cancer patients in the world was 1.1 million in that year, accounting for about 15% of the total number of new cancer cases, making it the second most common cancer in men worldwide. In the United States, the incidence of prostate cancer ranks first in all malignancies, with the second highest mortality rate, second only to lung cancer. Although the incidence of prostate cancer in China is much lower than that in western countries, it has shown a significant growth trend in recent years and ranks first among male urological tumors, and most of prostate cancer were diagnosed in the terminal stage.

The growth of the prostate cancer cells requires the supporting of androgens including testosterone. Therefore, the targeted treatment strategies for prostate cancer mainly focus on the synthesis of androgen and the binding to the androgen receptor thereof. For example, Enzalutamide, a prostate cancer drug marketed by the U.S. FDA in August 2012, is a small molecule androgen receptor antagonist, which finally inhibits the androgen receptor pathway by competitive inhibition of the binding of androgen to its receptor, thereby achieving the effect of treating castration-resistant prostate cancer.

Enzalutamide also shows some side effects in clinical studies, such as weakness or fatigue, lumbago, diarrhea, joint pain, hot flashes, tissue swelling, musculoskeletal pain, headache, upper respiratory tract infection, dizziness, spinal cord compression and cauda equina syndrome, muscle weakness, dyscoimesis, lower respiratory tract infection, hematuria, tingling, anxiety and hypertension and so on.

For the treatment of cancer, the drug combination is often used in the clinical practice to improve the treatment effect, for example, the combination of docetaxel and prednisone for use in the treatment of prostate cancer. However, people have met great setbacks when exploring new combination regimens. One of the typical examples is that although the combination of docetaxel and prednisone can treat prostate cancer (Tannock et al. N. Eng. J. Med. (2004), 351, 1502-1512), the combination regimen of docetaxel, , prednisone and lenalidomide failed in a Phase III clinical trial involving more than 1000 prostate cancer patients (Petrylak et al. Lancet Oncol. (2015) 16-4, 417-425*).* It should also be noted that, the results of several phase II clinical studies also indicated that the clinical efficacy of lenalidomide alone in the treatment of prostate cancer was not satisfying (Xing et al. Asian Pac. J. Cancer Prev. (2015) 16- 9, 3969-3972*).* Therefore, it has become an urgent technical problem to be solved in the art to explore combination regimens of anti-prostate cancer drugs (including Enzalutamide etc.) to improve the efficacy and reduce the toxic and side effect. US 2003/045552 A1 discloses isoindole-imide compounds that are potent inhibitors of the production of TNF-α and IL-1β, and stimulators of the production of IL-10, and T-cells, in mammals. US 2011/196150 A1 discloses 4'-arylmethoxy isoindoline compounds, and pharmaceutically acceptable salts for use in treating, managing, and preventing various diseases and disorders. US 2015/080419 A1 discloses 5-substituted quinzolinone derivatives and pharmaceutical compositions comprising the same compounds for use in treating, preventing and managing various disorders. US 2004/087546 A1 discloses 1-oxo-and 1,3 dioxo-2-(2,6-dioxopiperidin-3-yl) isoindolines substituted with amino in the benzo ring for use in treating cancers. US 2014/011774 A1 discloses pharmaceutical compositions comprising combination of SARM compounds and antiresorptive agents such as SERM compounds, including, inter-alia, Raloxifene, and uses thereof for treating osteoporosis and associated diseases. William D. Figg ET AL, "A Randomized Phase II Trial of Thalidomide, an Angiogenesis Inhibitor, in Patients with Androgen-independent Prostate Cancer", Clinical Cancer Research, United States, (20010701), page 1888, discloses that thalidomide, an antiangiogenesis agent, has some activity in patients with metastatic prostate cancer who have failed multiple therapies. FIGG W D ET AL, "A Double-Blind Randomized Crossover Study of Oral Thalidomide Versus Placebo for Androgen Dependent Prostate Cancer Treated With Intermittent Androgen Ablation", JOURNAL OF UROLOGY, LIPPINCOTT WILLIAMS & WILKINS, BALTIMORE, MD, US, vol. 181, no. 3, discloses that thalidomide can prolong progression-free survival in men with biochemically recurrent prostate cancer treated with limited androgen deprivation therapy. GLTLLEY J LET AL, "Kinetics of Serum Androgen Normalization and Factors Associated With Testosterone Reserve After Limited Androgen Deprivation Therapy for Nonmetastatic Prostate Cancer", JOURNAL OF UROLOGY, LIPPINCOTT WILLIAMS & WILKINS, BALTIMORE, MD, US, vol. 180, no. 4, discloses androgen deprivation therapy for nonmetastatic prostate cancer. CHADI NABHAN ET AL, "Lenalidomide Monotherapy in Chemotherapy-Naive, Castration-Resistant Prostate Cancer Patients: Final Results of a Phase II Study", CLINICAL GENITOURINARY CANCER, US, (20140201), vol. 12, no. 1, discloses that lenalidomide monotherapy demonstrates modest activity in chemotherapy-naive CRPC. WO 2011/050962 A1 discloses acid addition salts of lenalidomide and/or pomalidomide as well as to desirable polymorphic forms of lenalidomide hydrogen sulfate for the treatment of cancer, immune disorders and / or transplantation therapy. Jeanny B Aragon-Ching ET AL, "Thalidomide analogues as anticancer drugs", Recent patents on anti-cancer drug discovery, United Arab Emirates, (20070601), pages 167 - 174, discloses multiple thalidomide analogues including lenalidomide for use in treating solid tumor malignancies including prostate cancer, melanoma, and gliomas. CHADI NABHAN ET AL, "The Role of IMiDs Alone or in Combination in Prostate Cancer", CLINICAL GENITOURINARY CANCER, US, (20120901), vol. 10, no. 3, discloses different mechanisms that render prostate cancer castration resistant and elaborate on thalidomide and lenalidomide data in CRPC. ANDRESSA ARDIANI ET AL, "Androgen deprivation therapy sensitizes prostate cancer cells to T-cell killing through androgen receptor dependent modulation of the apoptotic pathway", ONCOTARGET, United States, (20141015), vol. 5, no. 19, discloses that the NAIP gene was significantly down-regulated in human prostate tumor cells treated *in vitro* and *in vivo* with enzalutamide. MICHAEL E. JUNG ET AL, "Structure-Activity Relationship for Thiohydantoin Androgen Receptor Antagonists for Castration-Resistant Prostate Cancer (CRPC)", JOURNAL OF MEDICINAL CHEMISTRY, (20100408), vol. 53, no. 7, discloses that a structure-activity relationship study was carried out on a series of thiohydantoins and their analogues which led to the discovery of a potential clinical candidate for the treatment of hormone refractory prostate cancer.

### Content of the present invention

The invention is set out in the appended set of claims.

The technical problem to be solved in the present invention is to improve the efficacy of the present anti-prostate cancer drugs (including Enzalutamide etc.).

The references to the methods of treatment by therapy or surgery or *in vivo* diagnosis methods in embodiments of this description are to be interpreted as references to compounds, pharmaceutical compositions and medicaments of the present invention for use in those methods.

In one aspect of the present invention, it provides a pharmaceutical composition, comprising: i) a benzoheterocyclic compound, a pharmaceutically acceptable salt thereof, or a stereoisomer thereof, and ii) one or more androgen receptor pathway modulator;
wherein the combination of the benzoheterocyclic compound and the androgen receptor pathway modulator is that: B001 and Enzalutamide, K001 and Enzalutamide, D107 and Enzalutamide, B001 and ARN-509, K001 and ARN-509, D107 and ARN-509, B001 and Galeterone, K001 and Galeterone, D107 and Galeterone, B001 and ODM-201, K001 and ODM-201, D108 and ODM-201, F001 and ODM-201, B002 and Enzalutamide, B003 and Enzalutamide, B004 and Enzalutamide, B005 and Enzalutamide, B006 and Enzalutamide, B002 and ARN-509, B003 and ARN-509, B004 and ARN-509, B005 and ARN-509, B006 and ARN-509, B001 and Enzalutamide and Galeterone, B001 and Enzalutamide and Abiraterone acetate, B001 and ARN-509 and Abiraterone acetate, B001 and ARN-509 and Galeterone, K001 and Enzalutamide and Galeterone, K001 and Enzalutamide and Abiraterone acetate, K001 and ARN-509 and Abiraterone acetate, K001 and ARN-509 and Galeterone;
wherein the structures of B001, B002, B003, B004, B005, B006, F001, K001, D107 and D108 are as follows: 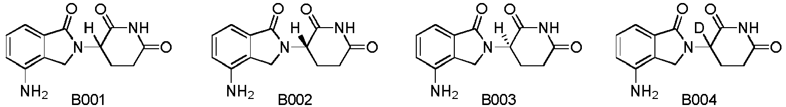 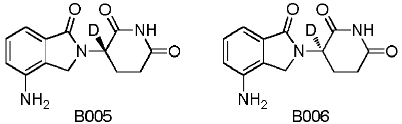 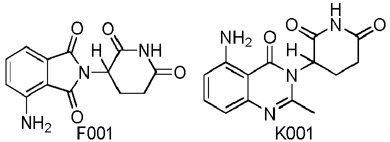 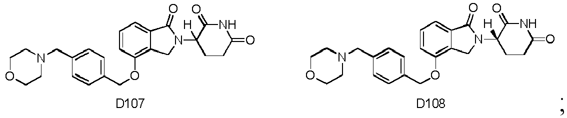 and
wherein
the structure ofARN-509 is 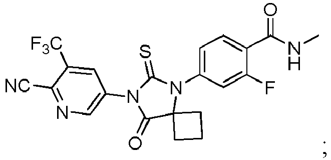
the structure of ODM-201 is

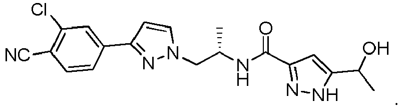

In an embodiment of the present invention, it provides the above-mentioned pharmaceutical composition for use in the prevention and/or treatment of prostate cancer.

In some embodiments of the present invention, in the above-mentioned pharmaceutical composition for use in the prevention and/or treatment of prostate cancer, the benzoheterocyclic compound and the androgen receptor pathway modulator are administered simultaneously, or in a sequential order, or separately.

In one aspect of the present invention, it provides a pharmaceutical composition comprising: i) a benzoheterocyclic compound, a pharmaceutically acceptable salt thereof, or a stereoisomer thereof, and ii) one or more androgen receptor pathway modulator and iii) a hormone compound;
wherein the benzoheterocyclic compound is B001, K001 or D101; and
wherein the combination of the androgen receptor pathway modulator and the hormone compound is prednisone and Abiraterone acetate, Enzalutamide and prednisone, ARN-509 and prednisone, Enzalutamide and Galeterone and prednisone, Enzalutamide and Abiraterone acetate and prednisone, ARN-509 and Galeterone and prednisone, ARN-509 and Abiraterone acetate and prednisone;
wherein the structures of B001, K001 and D101 are as follows: 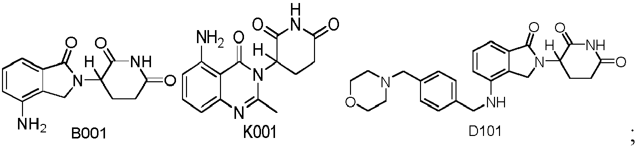 and
wherein
the structure ofARN-509 is

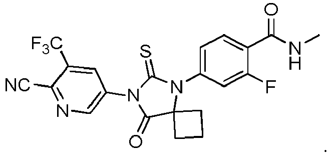

In another aspect of the present invention, it provides the above-mentioned pharmaceutical composition for use in the prevention and/or treatment of prostate cancer.

In some embodiments of the present invention, in the above-mentioned pharmaceutical composition for use in the prevention and/or treatment of prostate cancer, the benzoheterocyclic compound and the androgen receptor pathway modulator are administered simultaneously, or in a sequential order, or separately.

In the pharmaceutical composition, the use or the method of prevention and/or treatment of prostate cancer of the present invention, the mole ratio of the above-mentioned benzoheterocyclic compound and the androgen receptor pathway modulator, which may be selected in accordance with the conventional art, is preferably 1:0.0001-1:50, more preferably 1:0.0005-1:30 (1:0.0005, 1:0014, 1:0.004, 1:0.013, 1:0.04, 1:0.12, 1:0.33, 1:1, 1:3, 1:9, 1:27), even more preferably 1:0.1-1:10 .

In the pharmaceutical composition, the use or the method of prevention and/or treatment of prostate cancer of the present invention, the amount of the above-mentioned benzoheterocyclic compound and the androgen receptor pathway modulator is not particularly limited, which may be selected in accordance with the conventional art, for example, the amount of the above-mentioned benzoheterocyclic compound may be 0.01-300µM, preferably 0.05-200µM , more preferably 0.4-100µM (for example 100.00µM, 33.33µM, 11.11µM, 3.70µM, 1.23µM, 0.41µM); the amount of the androgen receptor pathway modulator may be 0.01-100 µM, preferably 0.05-50 µM, more preferably 0.05-30 µM, even more preferably 0.05-12 µM(for example 11.11 µM, 3.70 µM, 1.23 µM, 0.41 µM, 0.14 µM, 0.05 µM ), particularly preferably 0.1-10 µM.

In the pharmaceutical composition, the use or the method of prevention and/or treatment of prostate cancer of the present invention, when further comprising the hormonal compound, the amount of the hormonal compound is not particularly limited if the interaction between the above-mentioned benzoheterocyclic compound and the androgen receptor pathway modulator as described above is not affected; the mole ratio of the hormonal compound and the androgen receptor pathway modulator is preferably 1:0.01-1:100, more preferably 1:0.1-1:10 (for example 1:0.1, 1:1, 2:1, 1:10).

In the pharmaceutical composition, the use or the method of prevention and/or treatment of prostate cancer of the present invention, when further comprising the hormonal compound, the amount of the hormonal compound is not particularly limited if the interaction between the above-mentioned benzoheterocyclic compound and the androgen receptor pathway modulator as described above is not affected; the amount of the hormonal compound and the androgen receptor pathway modulator may be independently 0.01-100µM, preferably 0.05-50µM , more preferably 0.05-30µM, even more preferably 0.05-12µM(for example 11.11µM, 10µM, 5µM, 3.70µM, 2µM, 1.23µM, 1µM, 0.41µM, 0.14µM, 0.1µM, 0.05µM and so on), particularly preferably 0.1-10µM.

The pharmaceutical composition of the present invention may be formulated into any form for administration, including injection (intravenous), mucosal, oral administration (solid and liquid preparation), inhalation, ocular administration, rectal administration, topical or parenteral (infusion, injection, implantation, subcutaneous, vein, artery, intramuscular) administration. The pharmaceutical composition of the present invention can also be a controlled release or delayed release preparation. Examples of solid oral preparations include but not limited to powder, capsule, caplet, soft capsule, pill and tablet. Examples of liquid preparations for oral or mucosal administration include but not limited to suspension, emulsion, elixir and solution. Examples of preparations for topical administration include but not limited to emulsion, gel, ointment, cream, patch, paste, foam, lotion, drops or serum preparation. Examples of preparations for parenteral administration include but not limited to injection solution, dry preparation which can be dissolved or suspended in a pharmaceutically acceptable carrier, injection suspension and injection emulsion. Examples of other suitable preparations of the pharmaceutical composition, include but not limited to eye drops and other ophthalmic preparations; aerosol, such as nasal spray or inhalation; liquid dosage forms suitable for parenteral administration; suppository and pastille.

The pharmaceutical composition of the present invention may further comprises a pharmaceutically acceptable excipient, such as those widely used in drug manufacture field. The excipient is mainly used to provide a safe, stable and functionalized pharmaceutical composition, and can also provide a process which makes the active ingredients dissolved at a desired rate after the subject receives administration or promotes the effective absorbtion of the active ingredients after the subject is administered with the composition. The excipient can be an inert filler, or provide a certain function, such as stabilizing the overall pH value of the composition or preventing the degradation of the active ingredients of the composition. The pharmaceutically acceptable excipient may comprise one or more of the following excipients: binder, suspending agent, emulsifier, diluent, filler, granulating agent, adhesive, disintegrating agent, lubricant, anti-adhesive agent, glidant, wetting agent, gelling agent, absorption retarder, dissolution inhibitor, reinforcing agent, adsorbent, buffer, chelating agent, preservative, colorant, flavoring agent and sweetening agent. The pharmaceutically acceptable carrier can take a variety of forms depending on the form of preparation desired. For example, for liquid oral preparation, the suitable carriers and additives include water, glycols, oils, alcohols, flavoring agent, preservative, colorant, etc. As another illustrative example, for solid oral preparation, suitable carriers and additives include starch, sugar, diluent, granulation agent, lubricant, adhesive, disintegrating agent, etc. The pharmaceutically acceptable carriers or excipients usually should be non-toxic. The pharmaceutical composition of the present invention may comprise one or more suitable carrier(s)/excipient(s). The amount and type of the excipient vary depending on the requirements. A person skilled in the art can easily determine the appropriate carrier(s)/excipient(s) to be added to the pharmaceutical composition of the present invention based on the contents disclosed herein.

The pharmaceutical composition of the present invention can be prepared according to the disclosure using any method known to people skilled in the art. For example, the pharmaceutical composition of the present invention can be prepared by mixing one or more of the above-mentioned benzoheterocyclic compound, the pharmaceutically acceptable salt thereof, the stereoisomer thereof as well as the androgen receptor pathway modulator with the pharmaceutically acceptable carrier and, or by mixing one or more of the above-mentioned benzoheterocyclic compound, the pharmaceutically acceptable salt thereof, the stereoisomer thereof, the androgen receptor pathway modulator as well as the hormonal compound with the pharmaceutically acceptable carrier, according to conventional drug compounding technologies, which include but not limited to conventional mixing, dissolving, granulating, emulsifying, grinding, encapsulating, embedding or lyophilization.

In some embodiments, the pharmaceutical composition of the present invention relates to a controlled release preparation. As used herein, "controlled release preparation" refers to a preparation, wherein the therapeutic active ingredient of the pharmaceutical composition has a controlled release rate, or a specific delay to control the release site of the therapeutic active ingredient in the subject administered with the pharmaceutical composition. One controlled release preparation may comprise a controlled release agent, such as a sustained release agent (sustained release or delayed release) and a delayed release agent (delayed release).

As used herein, the terms "sustained release" and "delayed release" refer to prolonging the release of the therapeutic active ingredient from the pharmaceutical composition. As used herein, the term "delayed release" refers to that the therapeutic active ingredient releases from the pharmaceutical composition at a specific site or in a desired environment when the composition reaches the desired environment in the subject who has received administration or after a specific period of time since the subject receives administration.

As used herein, the terms "sustained release agent" and "delayed release agent" refer to a compound or an additive which controls the releasing of the therapeutic active ingredient from the composition, so as to make the release gradual and prolong the time of release. The sustained or delayed release agent may make the therapeutic active ingredient released within a specific period of time after the compostion was administered to a subject.

The "controlled release" from the pharmaceutical composition of the present invention can be achieved by a variety of conditions, including but not limited to pH, temperature, enzymes, water, or other physiological conditions or compounds. The pharmaceutical composition of the present invention may further comprise an enteric coating which controls the release of the active ingredient in the pharmaceutical composition, making it released gradually and continuously from the composition in a desired period of time, so that the active ingredient can play a therapeutic or preventive role for an extended period of time. The controlled release pharmaceutical composition may further comprise one or more of other therapeutic agents or medicaments as disclosed below.

One skilled in the art may be familiar with those appropriate controlled release preparations, sustained and delayed release agents based on the disclosed contents. Any composition described herein may be suitable for the controlled release preparation, such as tablet, capsule, soft capsule and caplet.

In the present invention, the term "active ingredient" refers to the active ingredient in the pharmaceutical composition of the present invention, that is, one or more of the above-mentioned benzoheterocyclic compound, the pharmaceutically acceptable salt thereof, the stereoisomer thereof and the androgen receptor pathway modulator, or one or more of the above-mentioned benzoheterocyclic compound, the pharmaceutically acceptable salt thereof, the stereoisomer thereof, the androgen receptor pathway modulator and the hormonal compound.

When the pharmaceutical composition of the present invention is administered to a subject for the purpose of treating or preventing a disease, disorder or condition, the active ingredient in the pharmaceutical composition may be administered by the same route or by a different route. The route of administration may be any route described herein, including but not limited to oral, inhalation, injection, ophthalmic, mucosal, rectal, emulsion, liposome, long-acting implant or sustained controlled release process. The specific route of administration will depend on the therapeutic agent itself and the preparation, as well as the disease, disorder or condition to be prevented or treated. According to the present disclosure, the skill level of an ordinary person skilled in the art is sufficient to determine the route of administration of other therapeutic agents. The active ingredient in the pharmaceutical composition of the present invention may be administered to the subject within a period of time (administration period) followed by a period of no administration of the compound (non-administration period). The administration period and non-administration period can be repeated for desired times. The desired length and times of the administration period or non-administration period will depend on the type and/or severity of the disease, disorder or condition being treated or prevented, as well as the sex, age, weight, and other parameters (e.g. the individual subject's biological, physical, and physiological status, etc.) of the individual subject. Each of the active ingredients in the pharmaceutical composition of the present invention may be administered simultaneously to the subject in a period of time and may also be administered to the subject sequentially in a period of time. According to the present disclosure, the skill level of an ordinary person skilled in the art is sufficient to determine the appropriate length and times of administration period and / or non-administration period.

The therapeutic method in the present invention comprises administering the pharmaceutical composition to a subject by any suitable processes, such as injection, mucosal, oral, inhalation, ocular, rectal, long-acting implant, liposome, emulsion or sustained release process.

One skilled in that art will understand that the therapeutically or prophylactically effective amount of the pharmaceutical composition of the present invention may vary with factors, for a specific subject, such as age, diet, health, etc., the symptom or disease to be treated or prevented, the severity of the disorder or condition, and the complications and types, and the preparations used etc. According to the disclosures in present invention, one skilled in the art can easily determine the desired therapeutically or prophylactically effective amount administered to the subject, so as to induce the desired biological or medical response in the subject.

The combined use of each of the active ingredients in the pharmaceutical composition according to the present invention may play a synergistic effect in the treatment or prevention of any disease, disorder or condition.

The pharmaceutical composition of the present invention may be administered to a subject for the treatment of the disease, disorder or condition of prostate cancer.

In one embodiment of the invention, the active ingredients in the pharmaceutical composition are administered to a subject simultaneously. In another embodiment, the active ingredients in the pharmaceutical composition are administered in a sequential order. In another embodiment, the active ingredients in the pharmaceutical composition are administered separately. The androgen receptor pathway modulator and/or the hormonal compound may be administered before, simultaneously with or after the administration of one or more of the above-mentioned benzoheterocyclic compound, the pharmaceutically acceptable salt thereof or the stereoisomer thereof.

In some embodiments, the therapeutically or prophylactically effective amount of the compound (herein referred as to one or more of the above-mentioned benzoheterocyclic compound, the pharmaceutically acceptable salt thereof orthe stereoisomer thereof, the androgen receptor pathway modulator, or the hormonal compound administered to each subject is from about 0.005 to about 1000 mg/day, from about 0.01 to about 500 mg/day, from about 0.01 to about 250 mg/day, from about 0.01 to about 100 mg/day, from about 0.1 to about 100 mg/day, from about 0.5 to about 100 mg/day, from about 1 to about 100 mg/day, from about 0.01 to about 50 mg/day, from about 0.1 to about 50 mg/day, from about 0.5 to about 50 mg/day, from about 1 to about 50 mg/day, from about 0.02 to about 25 mg/day, or from about 0.05 to about 10 mg/day.

In some embodiments, the therapeutically or prophylactically effective amount (herein referred as to the therapeutically or prophylactically effective amount of one or more of the above-mentioned benzoheterocyclic compound, the pharmaceutically acceptable salt thereof or the stereoisomer thereof, the androgen receptor pathway modulator, or the hormonal compound is about 0.01, about 0.05, about 0.1, about 0.2, about 0.3, about 0.4, about 0,5, about 0.6, about 0.8, about 1, about 2, about 5, about 10, about 15, about 20, about 25, about 30, about 40, about 45, about 50, About 60, about 70, about 80, about 90, about 100, about 150, about 200, about 250, about 300, about 350, about 400, about 450, about 500, about 550, about 600, about 650, about 700 About 750, about 800, about 850, about 900, or about 1000 mg/day/subject.

In another embodiment, the therapeutically or prophylactically effective amount of the androgen receptor pathway modulator or the hormone compound in the pharmaceutical composition of the present invention may be lower than the effective amount when the above-mentioned benzoheterocyclic compound, the pharmaceutically acceptable salt thereof or the stereoisomer thereof of the present invention is not administered.

In the present invention, the amount of the compound administered, the therapeutically or prophylactically effective amount, the dosage, the starting dosage and the like are all referred to the amount of a specific compound, for example, a specific above-mentioned benzoheterocyclic compound, a pharmaceutically acceptable salt thereof or a stereoisomer thereof, a specific androgen receptor pathway modulator or a specific hormone, rather than a combination of multiple compounds.

In the present invention, the therapeutically or prophylactically effective amount of the androgen receptor pathway modulator or the hormone compound in the method is well known to people skilled in the art, and the guidance for administration can be found in the patents and published patent applications cited herein, and Wells et al, eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000) and other medical literatures. However, an ordinary person skilled in the art is well-qualified to determine the optimal dose range of the other therapeutic agents.

The term "androgen receptor pathway modulator" in the present invention comprises androgen inhibitor, androgen receptor inhibitor, androgen biosynthesis inhibitor and other drugs that affect the androgen receptor pathway.

As used herein, when referring to a specific salt, composition, and excipient etc. as "pharmaceutical acceptable", it means that the salt, the composition, the excipient etc. are generally non-toxic, safe, and suitable for use in a subject, preferably a mammalian subject, more preferably a human subject.

The term "pharmaceutically acceptable salt" herein refers to a pharmaceutically acceptable organic or inorganic salt. Examples of the salt include but are not limited to: sulfate, citrate, acetate, oxalate, chloride, bromide, iodide, nitrate, hydrosulfate, phosphate, acid phosphate, isonicotinic acid salt, lactate, salicylic acid salt, acid citrate, tartrate, oleate, tannate, pantothenate, bitartrate, ascorbate, succinate, maleate, gentisinate, fumarate, gluconate, glucuronate, saccharate, formate, benzoate, glutamate, methane sulfonate, ethane sulfonate, benzene sulfonate, p-toluene sulfonate, and embonate (i.e. 1-1-methylene-bis(2-hydroxy-3-naphthoate)). The compounds of the present invention may form pharmaceutically acceptable salts with various amino acids. Suitable alkali salts include but are not limited to, aluminum salt, calcium salt, lithium salt, magnesium salt, potassium salt, sodium salt, zinc salt, bismuth salt and diethanolamine salt. For a review of the pharmaceutically acceptable salts, see Handbook of Pharmaceutical Salts: Properties, Selection, and Use (P. Heinrich Stahl and Camille G. Wermuth, ed., Wiley-VCH, 2002)

As used herein, the term "metabolite" refers to an active substance produced by changes in chemical structure that a drug molecule undergoes in vivo, the active substance is generally a derivative of the aforementioned drug molecule, and can also be chemically modified.

As used herein, the term "polymorph" refers to one or more crystal structures formed by the different arrangement of molecules in the lattice space when crystallized.

As used herein, the term "co-crystal" refers to a multi-component system comprising one or more API (active pharmaceutical ingredient) molecules and one or more object (or ligand) molecules. In the co-crystal, API molecules and object (or ligand) molecules exist as solids at room temperature when they are used as their pure form alone (in order to distinguish co-crystal from solvate or hydrate). From this particular definition, salts in which significant or complete proton exchange occurs between API molecules and guest molecules are excluded. In the co-crystal, API and ligands interact through hydrogen bonds and other possible non-covalent interactions. It is noted that the co-crystal itself may form solvates, including hydrates. The object (or ligand) refers to other physiologically acceptable acids, bases or non-ionic compounds.

As used herein, the term "solvate" refers to a crystal form of the above-mentioned benzoheterocyclic compound, the pharmaceutically acceptable salt, the polymorph, theco-crystal, the stereoisomer, the isotopic compound, the metabolite or the prodrug thereof, which further comprises one or more solvent molecule(s) incorporated into the crystal structure. The solvate may include a stoichiometric amount or a non-stoichiometric amount of solvent, and the solvent molecule in the solvent may exist in an ordered or non-ordered arrangement. The solvate containing a non-stoichiometric amount of solvent molecules may be obtained by the loss of at least one solvent molecule (but not all) from the solvate. In a particular embodiment, a solvate refers to a hydrate, which means the crystal of the compound further comprises water molecules, with water molecules as the solvent.

As used herein, the term "prodrug" refers to a derivative of the compound comprising a biologically reactive functional group such that the biological reactive functional group can be cleaved from the compound or react in other ways to give the compound under biological conditions (in vivo or in vitro). Usually, the prodrug is inactive, or at least has lower activity than the compound itself, such that the compound exhibit its activity until it is cleaved from the biologically reactive functional group. The biologically reactive functional group can be hydrolyzed or oxidized under biological conditions to give the compound. For instance, the prodrug may contain a biologically hydrolysable group. Examples of the biologically hydrolysable group include, but are not limited to: a biologically hydrolysable phosphate, a biologically hydrolysable ester, a biologically hydrolysable amide, a biologically hydrolysable carbonic ester, a biologically hydrolysable carbamate and a biologically hydrolysable ureide. For a review of the prodrug, see, for example, J. Rautio et al., Nature Reviews Drug Discovery (2008) 7, 255-270 and Prodrugs: Challenges and Rewards (V. Stella et al. ed., Springer, 2007).

The above-mentioned benzoheterocyclic compound, the pharmaceutically acceptable salt or the stereoisomer of the present invention, can contain one or more asymmetric centers ("stereoisomer"). As used herein, the term "stereoisomer" refers to all stereoisomers including enantiomers, diastereoisomers, epimers, endo-exo isomers, atropisomers, regioisomers, cis- and trans-isomers. The "stereoisomer" herein also includes "pure stereoisomer" and "enriched stereoisomer" or "racemic isomer" of the various aforementioned stereoisomers. These stereoisomers can be prepared according to an asymmetric synthesis process, or separated, purified and enriched by a chiral separation process (including but not limited to thin layer chromatography, rotating chromatography, column chromatography, gas chromatography, high pressure liquid chromatography, etc.), and can also be obtained through chiral separation by means of bonding (chemical binding etc.) or salifying (physical binding etc.) with other chiral compound(s). The term "pure stereoisomer" herein refers to a stereoisomer of the compound with the mass content of no less than 95% relative to other stereoisomers of the compound. The term "enriched stereoisomer" herein refers to a stereoisomer of the compound with the mass content of no less than 50% relative to other stereoisomers of the compound. The term "racemic isomer" herein refers to a stereoisomer of the compound with the mass content equal to that of other stereoisomers of the compound.

As used herein, D represents deuterium-enriched hydrogen, and H represents non-deuterium-enriched hydrogen. "Deuterium-enriched" compound means that abundance of deuterium at any relevant site in the above-mentioned benzoheterocyclic compound, a pharmaceutically acceptable salt thereof, a solvate thereof, a polymorph thereof, a co-crystal thereof, a stereoisomer thereof, an isotope compound thereof, a metabolite thereof or a prodrug thereof is greater than its natural abundance at that site (0.0156%). So, in the "deuterium-enriched" compounds, the abundance of deuterium at any of its related sites may be in the range of 0.0156% to 100%. An example of a process for obtaining deuterium-enriched compounds is to exchange hydrogen with deuterium or to synthesize the compound from deuterium-enriched starting material.

Based on the general knowledge in the art, the symbol H may be omitted in the non-deuterium-enriched site. "Non-deuterium enriched" refers to hydrogen in nature, i.e., in the form of isotopic mixture of H (hydrogen or protium), D (²H or deuterium) and T (³H or tritium).

The term "isotopic compound" used herein refers to the above-mentioned benzoheterocyclic compound, the pharmaceutically acceptable salt, the solvate, the polymorph, the co-crystal, the stereoisomer, the isotopic compound, the metabolite or the prodrug thereof containing one or more atomic isotope(s) with natural or non-natural abundance. Atomic isotopes with non-natural abundance include, but are not limited to: deuterium (²H or D), tritium (³H or T), iodine-125 (¹²⁵I), phosphorus-32 (³²P), carbon-13 (¹³C) or carbon-14 (¹⁴C). The aforementioned isotopic compound can also be used as a therapeutic or diagnostic agent (i.e., internal developing agent) or a research tool. All the isotopic variants of the compound of the present invention, whether radioactive or not, are included in the scope of the present invention.

The term "isotope enriched" used herein refers to the above-mentioned benzoheterocyclic compound, the pharmaceutically acceptable salt, the solvate or the stereoisomer thereof containing one or more atomic isotope(s) with non-natural abundance. The term "isotope enriched" also refers to the above-mentioned benzoheterocyclic compound, the pharmaceutically acceptable salt or the stereoisomer, the isotopic compound thereof containing at least one isotopic atom with non-natural abundance.

As used herein, the term "subject" refers to any animal to be treated or treated with the compound or the composition according to the embodiments of the present invention, preferably mammal, and most preferably human. The term "mammal" used herein includes any mammal. Examples of mammals include but are not limited to cattle, horse, sheep, pig, cat, dog, mouse, rat, rabbit, guinea pig, monkey, human and the like, most preferably human.

In an embodiment, the terms "treat" and "treating" refers to an improvement, prevention or reversal of a disease or condition or at least one of identifiable symptoms thereof, such as treating cancer by reducing or stabilizing the symptoms of the cancer or the condition. In another embodiment, "treat" or "treating" refers to an improvement, prevention or reversal of at least one measurable body parameter of a disease or condition which is being treated, but may not be identified in mammal. However, in another embodiment, the term "treat" or "treating" refers to slowing the progression of a disease or condition, in physical, such as stabilizing identifiable symptoms, or in physiological, such as stabilizing physical parameters, or in both. In another embodiment, the term "treat" or "treating" refers to delaying the development of a disease or symptom.

In some embodiments, the pharmaceutical composition is administered for a prevention purpose. As used herein, "prevent" or "preventing" refers to a reduction in a risk of obtaining a given disease or condition. In a preferred embodiment, the designated pharmaceutical composition is administered for a prevention purpose to a subject, such as a subject with family history or tendency of cancer or autoimmune disease.

As used herein, "therapeutically effective amount" refers to an amount of the compound or the composition (which is sought by researchers, veterinarians, physicians, or other clinicians) that can cause a biological or medical response in a tissue system, an animal or a person, which may include relieving symptoms of the disease or symptom which is being treated. In a preferred embodiment, the therapeutically effective amount is an amount which is enough to effectively treat, improve or prevent cancer, condition or undesirable angiogenesis.

The term "prophylactically effective amount" refers to an amount of the active compound or medicament (sought by researchers, veterinarians, physicians or other clinicians) that can inhibit the development of a disease in a subject. A prophylactically effective amount of the compound refers to an amount of the therapeutic agent used alone or in combination with other active compound, which can provide a therapeutic benefit for treating or preventing the disease, condition or disorder.

Each preferred conditions aforementioned can be combined randomly without departing from the common knowledge in the art thereby forming various preferred embodiments of the present invention.

Unless otherwise specified, the singular form of the term used herein, "a" or "an", also includes a plural meaning.

Unless otherwise specified, the term "or" or "and" used herein refers to "and/or".

Unless defined otherwise, all technical and scientific terms used herein have the same meaning as commonly understood by an ordinary person skilled in the art to which this invention belongs. Otherwise, the meaning of certain terms used herein has the meaning set forth in this description.

In the present invention, the structures of the androgen receptor pathway modulator and the hormone comoound are as follows:

| | |
|---|---|
| Enzalutamide | 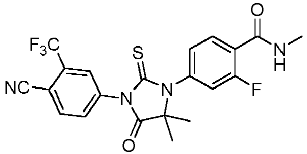 |
| ARN-509 (Apalutamide) | 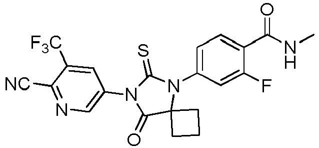 |
| ODM-201 (BAY-1841788) | 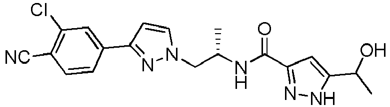 |
| VT-464 | 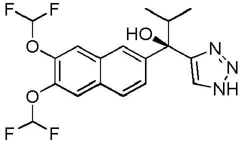 |
| Orteronel (TAK-700) | 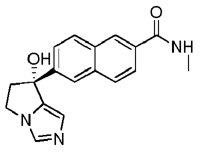 |
| EPI-001 | 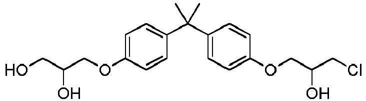 |
| Andarine (GTx-007) | 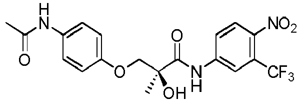 |
| RD162 | 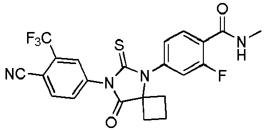 |
| BMS-641988 | 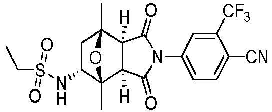 |
| CH5137291 | 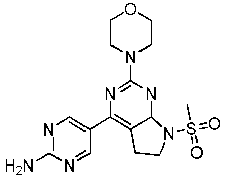 |
| Flutamide | 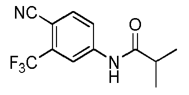 |
| Hy droxyflutami de | 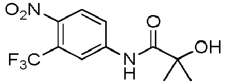 |
| RU58642 | 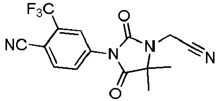 |
| LG120907 | 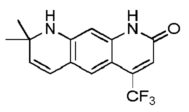 |
| LG105 | 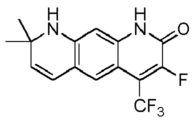 |
| Galeterone (TOK-001) | 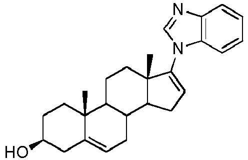 |
| Abiraterone acetate | 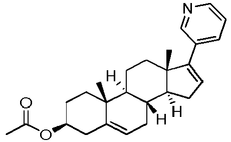 |
| Abiraterone | 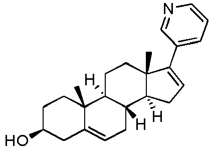 |
| Spironolactone | 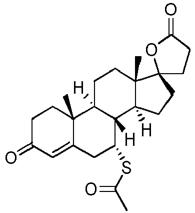 |
| MK-2866 (GTx-024) | 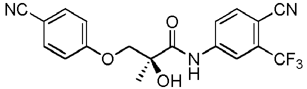 |
| AZD3514 | 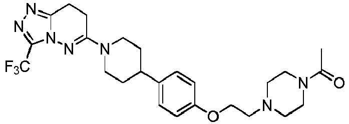 |
| Dehydroepiandrosterone | 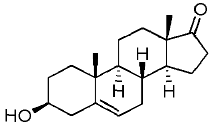 |
| Epiandrosterone | 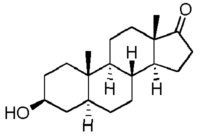 |
| Cyproterone Acetate | 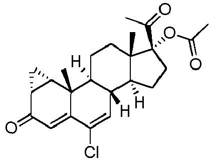 |
| Prednisone | 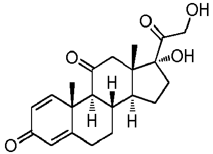 |
| ORM-15341 | 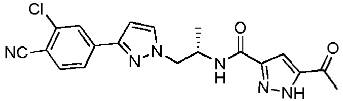 |
| Degarelix | 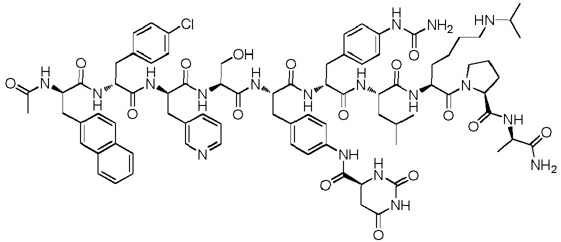 |
| Goserelin acetate | 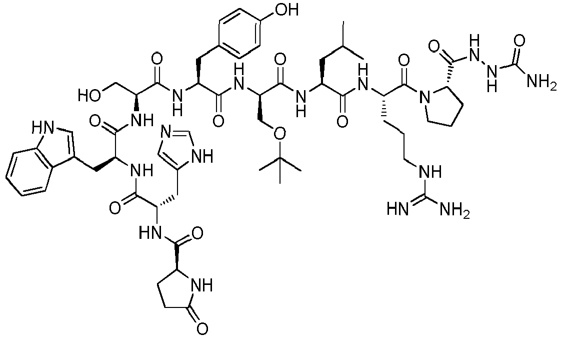 |
| Leuprolide acetate | 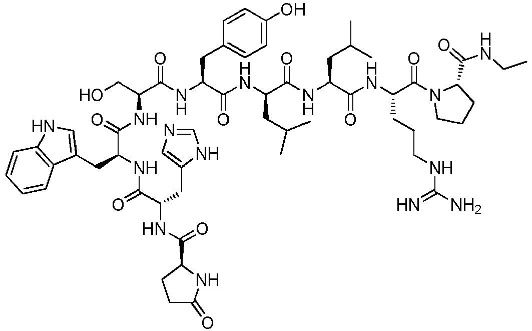 |

The reagents used in the present invention are all commercially available. The above-mentioned benzoheterocyclic compound and the androgen receptor pathway modulator in the present invention may be obtained commercially or prepared by people skilled in the art according to synthetic methods well known in the art, or readily synthesized according to the published literatures or patents, such as WO9803502, WO2010056344, WO2012079022, WO2012015986, WO2011100380, WO2014116573, WO2008039489, WO2014110558, WO2014039421, WO2006124118 and so on.

Each preferred conditions aforementioned can be combined randomly without departing from the common knowledge in the art thereby forming various preferred embodiments of the present invention.

The positive effect of the present invention is that the pharmaceutical composition of the present invention can inhibit the growth of prostate cancer cells more effectively.

### Detailed description of the embodiments

The following combinations: B001 and Enzalutamide, K001 and Enzalutamide, D107 and Enzalutamide, B001 and ARN-509, K001 and ARN-509, D107 and ARN-509, B001 and Galeterone, K001 and Galeterone, D107 and Galeterone, B001 and ODM-201, K001 and ODM-201, D108 and ODM-201, F001 and ODM-201, B002 and Enzalutamide, B003 and Enzalutamide, B004 and Enzalutamide, B005 and Enzalutamide, B006 and Enzalutamide, B002 and ARN-509, B003 and ARN-509, B004 and ARN-509, B005 and ARN-509, B006 and ARN-509, B001 and Enzalutamide and Galeterone, B001 and Enzalutamide and Abiraterone acetate, B001 and ARN-509 and Abiraterone acetate, B001 and ARN-509 and Galeterone, K001 and Enzalutamide and Galeterone, K001 and Enzalutamide and Abiraterone acetate, K001 and ARN-509 and Abiraterone acetate, K001 and ARN-509 and Galeterone, B001 and prednisone and Abiraterone acetate, B001 and Enzalutamide and prednisone, B001 and ARN-509 and prednisone, B001 and Enzalutamide and Galeterone and prednisone, B001 and Enzalutamide and Abiraterone acetate and prednisone, B001 and ARN-509 and Galeterone and prednisone, B001 and ARN-509 and Abiraterone acetate and prednisone, K001 and prednisone and Abiraterone acetate, K001 and Enzalutamide and prednisone, K001 and ARN-509 and prednisone, K001 and Enzalutamide and Galeterone and prednisone, K001 and Enzalutamide and Abiraterone acetate and prednisone, K001 and ARN-509 and Galeterone and prednisone, K001 and ARN-509 and Abiraterone acetate and prednisone, D101 and prednisone and Abiraterone acetate, D101 and Enzalutamide and prednisone, D101 and ARN-509 and prednisone, D101 and Enzalutamide and Galeterone and prednisone, D101 and Enzalutamide and Abiraterone acetate and prednisone, D101 and ARN-509 and Galeterone and prednisone, D101 and ARN-509 and Abiraterone acetate and prednisone in the effect embodiment 1 or effect embodiment 2 are according to the invention, all the other combinations in the effect embodiment 1 and effect embodiment 2 are reference combinations and are not according to the invention.

### Effect embodiment 1 CTG cell proliferation assay

In vitro test of the inhibition effect of the compound in combination with androgen receptor pathway modulators and the like on the prostate cancer cell proliferation.

Inhibition effect of the compounds such as B001, K001, D101, D107, D108, F001, B002, B003, B004, B005, B006 and the like alone or in combination with androgen receptor pathway modulators on the prostate cancer cell proliferation were tested on Vcap cells (androgen receptor (+) prostate cancer cells) (ATCC, catalogue number CRL-2876) The specific experimental operation was as follows: 5×10³ Vcap cells per well were inoculated into 96-well plates with transparent bottom and white wall (Corning, catalogue number CLS3903) containing the specific medium, and were cultured in a 37 °C, 5% CO₂ incubator for 24 hours. The tested compounds and the androgen receptor pathway modulators were prepared to a 150mM stocking solution with DMSO (Sigma, catalogue number 276855), diluted with culture medium to the desired concentrations (the final concentration of DMSO is 0.2%), and then added to each well, 2 wells/concentration, followed by being incubated in a 37 °C, 5% CO₂ incubator for 5 days. The tested compounds were used alone or in combination with other androgen receptor pathway modulators respectively. The combination drugs were: Enzalutamide (Kangpu Biopharmaceuticals, Ltd.), ARN-509 (Selleck, catalogue number S2840), Abiraterone acetate (Selleck, catalogue number S2246), Galeterone (Selleck, catalogue number S2803), ODM-201 (Kangpu Biopharmaceuticals, Ltd.) or Prednisone (Selleck, catalogue number S1622). The concentration setting of each drug was shown in the following tables of experimental results. After that, 100 µl of CellTiter-Glo^{®} cell viability assay reagent (Promega, catalogue number G7570) was added to each well and mixed well on a vibrator for 10 minutes to induce cell lysis. The 96-well plate was placed at room temperature for 10 minutes, so as to stabilize its luminescence signal. A white bottom membrane was pasted on the bottom of the plate and the plate was tested using EnSpire. The data was processed by Graphpad/Prism and Calcusyn software to calculate the average cell proliferation inhibition rate or survival rate for each compound or the synergism index of the drug combination, and the specific experimental results were shown in Tables 1-10.

**Table 1. Vcap cell proliferation inhibition rate:the combination of B001 and Enzalutamide**

| | | B001 (µM) | | | | | | |
|---|---|---|---|---|---|---|---|---|
| | | **100.00** | **33.33** | **11.11** | **3.70** | **1.23** | **0.41** | **0.00** |
| Enzalutamide (µM) | **11.11** | 59.3% | 59.0% | 56.9% | 55.4% | 54.3% | 52.5% | 36.7% |
| | **3.70** | 60.1% | 61.3% | 61.1% | 59.6% | 57.6% | 55.1% | 37.4% |
| | **1.23** | 57.2% | 56.7% | 58.9% | 57.1% | 54.8% | 52.6% | 28.7% |
| | **0.41** | 44.4% | 52.0% | 52.6% | 50.2% | 50.7% | 48.1% | 28.1% |
| | **0.14** | 34.1% | 39.4% | 41.2% | 39.8% | 38.6% | 37.9% | 25.0% |
| | **0.05** | 27.4% | 32.1% | 31.6% | 30.0% | 30.8% | 30.2% | 17.2% |
| | **0.00** | 19.6% | 22.0% | 20.9% | 21.4% | 20.7% | 18.7% | 6.5% |

| | | | | | | | | |
|---|---|---|---|---|---|---|---|---|
| Noes of table 1: The cell proliferation inhibition rate (%) was measured after processing the Vcap cells with different concentrations of B001 (0.41-100µM) and Enzalutamide (0.05-11.11µM) alone or in combination for 5 days. The effect of drug combination was outstanding, for example, theinhibition rate on Vcap cells was 20.7% when B001 was used alone (concentration of 1.23 µM), the inhibition rate on Vcap cells was 28.7% when Enzalutamide was used alone (concentration of 1.23 µM), and the inhibition rate on Vcap cells was 54.8% when the two were combined (1.23 µM B001 and 1.23 µM Enzalutamide). | | | | | | | | |

**Table 2. Synergism index of the combination of B001 and Enzalutamide (carrying out synergism analysis on the experimental data of the drug combination in table 1**

| | | B001 (µM) | | | | | |
|---|---|---|---|---|---|---|---|
| | | **100.00** | **33.33** | **11.11** | **3.70** | **1.23** | **0.41** |
| Enzalutamide (µM) | **11.11** | **0.012** | **0.012** | **0.02** | **0.028** | **0.035** | **0.053** |
| | **3.70** | **0.003** | **0.002** | **0.003** | **0.004** | **0.006** | **0.01** |
| | **1.23** | **0.002** | **0.002** | **0.001** | **0.002** | **0.004** | **0.006** |
| | **0.41** | **0.012** | **0.002** | **0.002** | **0.003** | **0.003** | **0.005** |
| | **0.14** | **0.043** | **0.012** | **0.008** | **0.011** | **0.015** | **0.017** |
| | **0.05** | **0.088** | **0.025** | **0.029** | **0.044** | **0.036** | **0.042** |

| | | | | | | | |
|---|---|---|---|---|---|---|---|
| Notes of the synergism index of drug combination: <0.1: very strong synergism; 0.1-0.3: strong synergism; 0.3-0.7: synergism; 0.7-0.85: mild synergism; 0.85-0.90: slight synergism; 0.90-1.10: approximately additive action; 1.10- 1.20: slight antagonism; 1.20-1.45: mild antagonism; 1.45-3.3: antagonism; 3.3-10: strong antagonism; >10: very strong antagonism. Notes of table 2: The synergism analysis was carried out on the experimental data of the drug combination of B001 and Enzalutamide in table 1 to give the data in Table 2 which showed a strong synergism when the two drugs are used in combination. | | | | | | | |

**Table 3: Survival rate of Vcap cells: tested compound alone, tested compound in combination with Enzalutamide or ARN-509**

| Tested compound | Tested compound alone | | | In combination with 1 µM Enzalutamide | | | In combination with 1 µM ARN-509 | | |
|---|---|---|---|---|---|---|---|---|---|
| Concentration of the tested compound | 10µM | 1.0µM | 0.1µM | 10µM | 1.0µM | 0.1µM | 10µM | 1.0µM | 0.1µM |
| Enzalutamide | 52.3% | 54.0% | 74.5% | | | | | | |
| ARN-509 | 48.9% | 52.2% | 81.8% | | | | | | |
| B001 | 81.9% | 82.8% | 92.0% | 33.7% | 35.6% | 40.3% | 33.5% | 34.7% | 42.0% |
| K001 | 61.4% | 65.8% | 85.1% | 35.1% | 36.4% | 42.4% | 33.5% | 36.0% | 43.6% |
| D107 | 69.3% | 77.2% | 85.3% | 32.3% | 35.0% | 37.3% | 34.2% | 36.3% | 37.0% |

| | | | | | | | | | |
|---|---|---|---|---|---|---|---|---|---|
| Notes of table 3: The effect of the drug combination was outstanding, for example, the cell survival rates were 82.8%, 65.8%, 77.2%, 54.0% and 52.2% when B001, K001, D107, enzalutamide and ARN-509 were used alone respectively at 1.0 µM. The cell survival rates were 35.6%, 36.4% and 35.0% when 1.0 µM B001, K001, or D107 was used in combination with 1.0 µM enzalutamide respectively. The cell survival rates were 34.7%, 36.0% and or 36.3% when 1.0 µM B001, K001, or D107 was used in combination with 1.0 µM ARN-509 respectively. | | | | | | | | | |

**Table 4: Survival rate of Vcap cells: tested compound alone, tested compound in combination with Galeterone or Abiraterone acetate**

| Tested compounds | Tested compounds alone | | | In combination with 1 µM Galeterone | | | In combination with 1 µM Abiraterone acetate | | |
|---|---|---|---|---|---|---|---|---|---|
| Concentration of the tested compounds | 10µM | 1µM | 0.1µM | 10µM | 1µM | 0.1µM | 10µM | 1µM | 0.1µM |
| Enzalutamide | 52.3% | 54.0% | 74.5% | 50.9% | 52.4% | 59.5% | 51.7% | 50.7% | 69.7% |
| B001 | 81.9% | 82.8% | 92.0% | 42.8% | 43.3% | 51.1% | 62.9% | 68.0% | 78.5% |
| K001 | 61.4% | 65.8% | 85.1% | 39.4% | 41.3% | 49.1% | 48.1% | 53.1% | 70.4% |
| D107 | 69.3% | 77.2% | 85.3% | 40.0% | 42.4% | 43.9% | 59.6% | 61.7% | 66.0% |
| Galeterone | 19.3% | 62.8% | 95.9% | / | / | / | / | / | / |
| Abiraterone acetate | 31.4% | 91.7% | 106.3% | / | / | / | / | / | / |

| | | | | | | | | | |
|---|---|---|---|---|---|---|---|---|---|
| Notes of table 4: The effect of the drug combination was outstanding, for example, the cell survival rates were 82.8%, 65.8%, 77.2%, 62.8% and 91.7% when B001, K001, D107, Galeterone and Abiraterone acetate were used alone respectively at 1.0 µM. The cell survival rates were 43.3%, 41.3% and 42.4% when 1.0 µM B001, K001, or D107 was used in combination with 1.0 µM Galeterone respectively. The cell survival rates were 68.0%, 53.1% and 61.7% when 1.0 µM B001, K001, or D107 was used in combination with 1.0 µM Abiraterone acetate respectively. | | | | | | | | | |

**Table 5: Survival rate of Vcap cells: tested compound alone, tested compound in combination with ODM-201**

| Tested compounds | Tested compounds alone | | | In combination with 1µM ODM-201 | | |
|---|---|---|---|---|---|---|
| Concentration of the tested compound | 10µM | 1.0µM | 0.1µM | 10µM | 1.0µM | 0.1µM |
| ODM-201 | 75.4% | 69.0% | 90.7% | | | |
| K001 | 51.0% | 59.7% | 79.2% | 35.2% | 40.4% | 53.5% |
| B001 | 72.3% | 75.3% | 87.7% | 37.5% | 41.4% | 52.7% |
| D108 | 58.5% | 67.3% | 73.0% | 32.8% | 40.1% | 42.7% |
| F001 | 55.7% | 59.9% | 79.7% | 37.3% | 40.9% | 49.4% |

| | | | | | | |
|---|---|---|---|---|---|---|
| Notes of table 5: The effect of the drugs combination was outstanding, for example, the cell survival rates were 69.0%, 59.7%, 75.3%, 67.3% and 59.9% respectively when ODM-201, K001, B001, D108, and F001 were used alone at 1.0 µM. The cell survival rates were 40.4%, 41.4%, 40.1% and 40.9% when 1.0 µM K001, B001, D108 or F001 was used in combination with 1.0 µM ODM-201 respectively. | | | | | | |

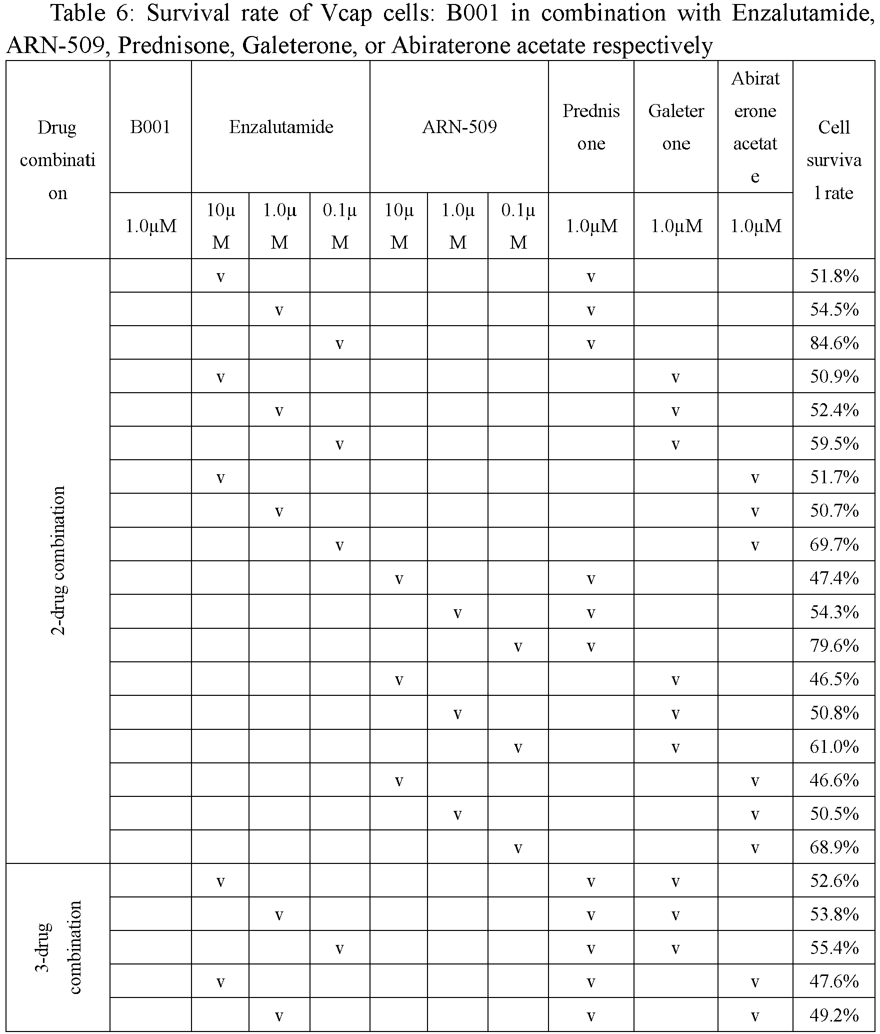

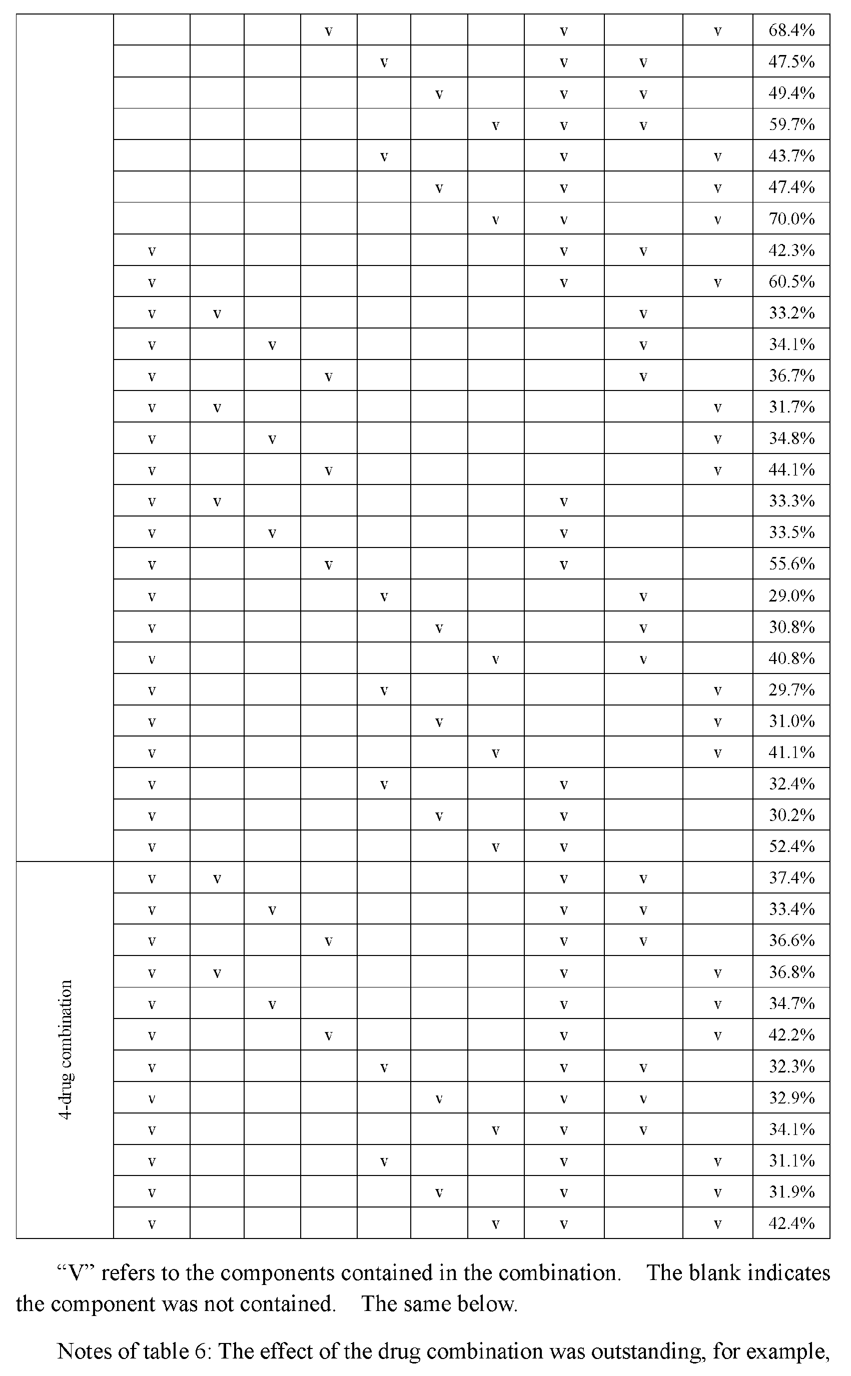

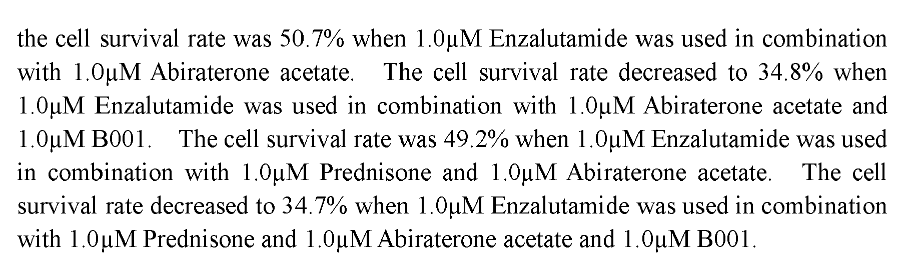

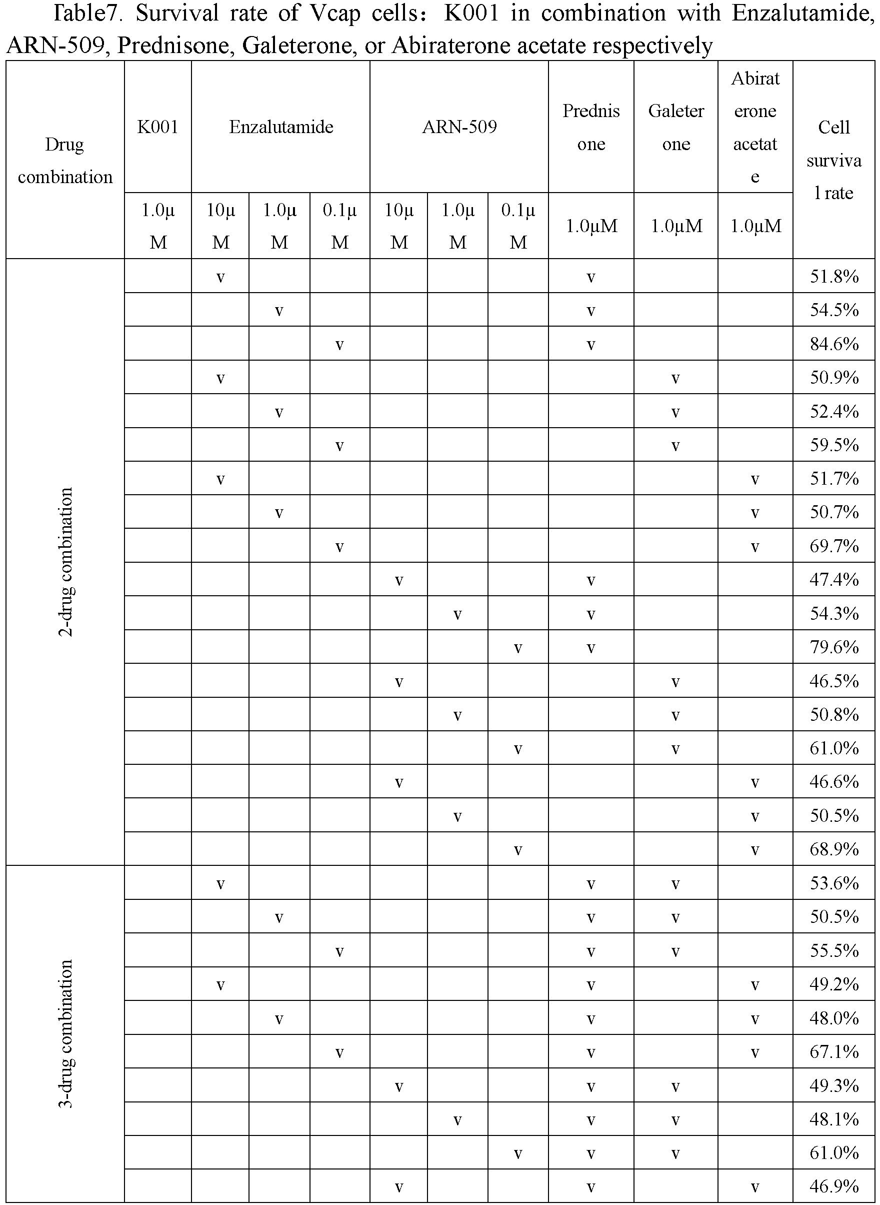

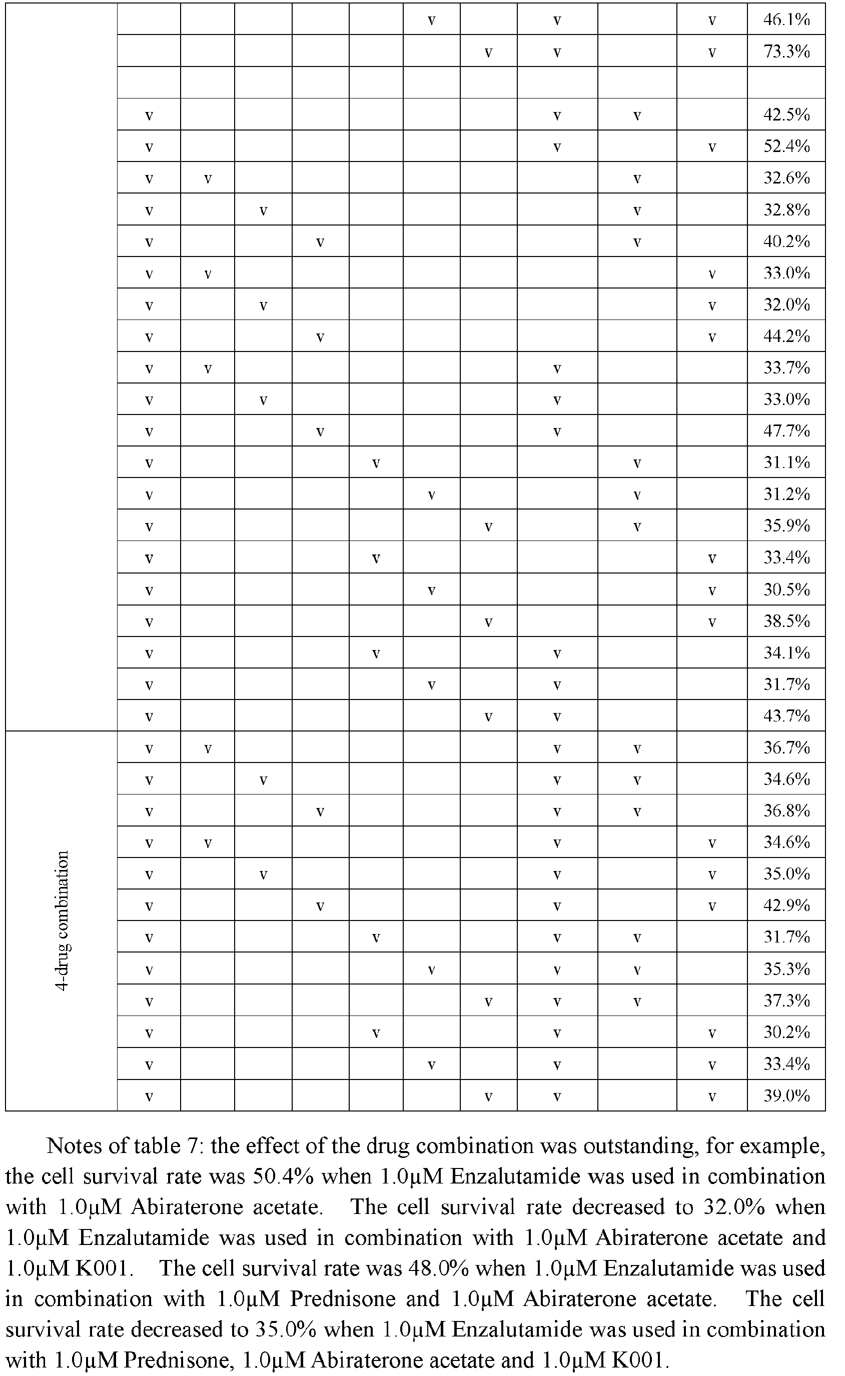

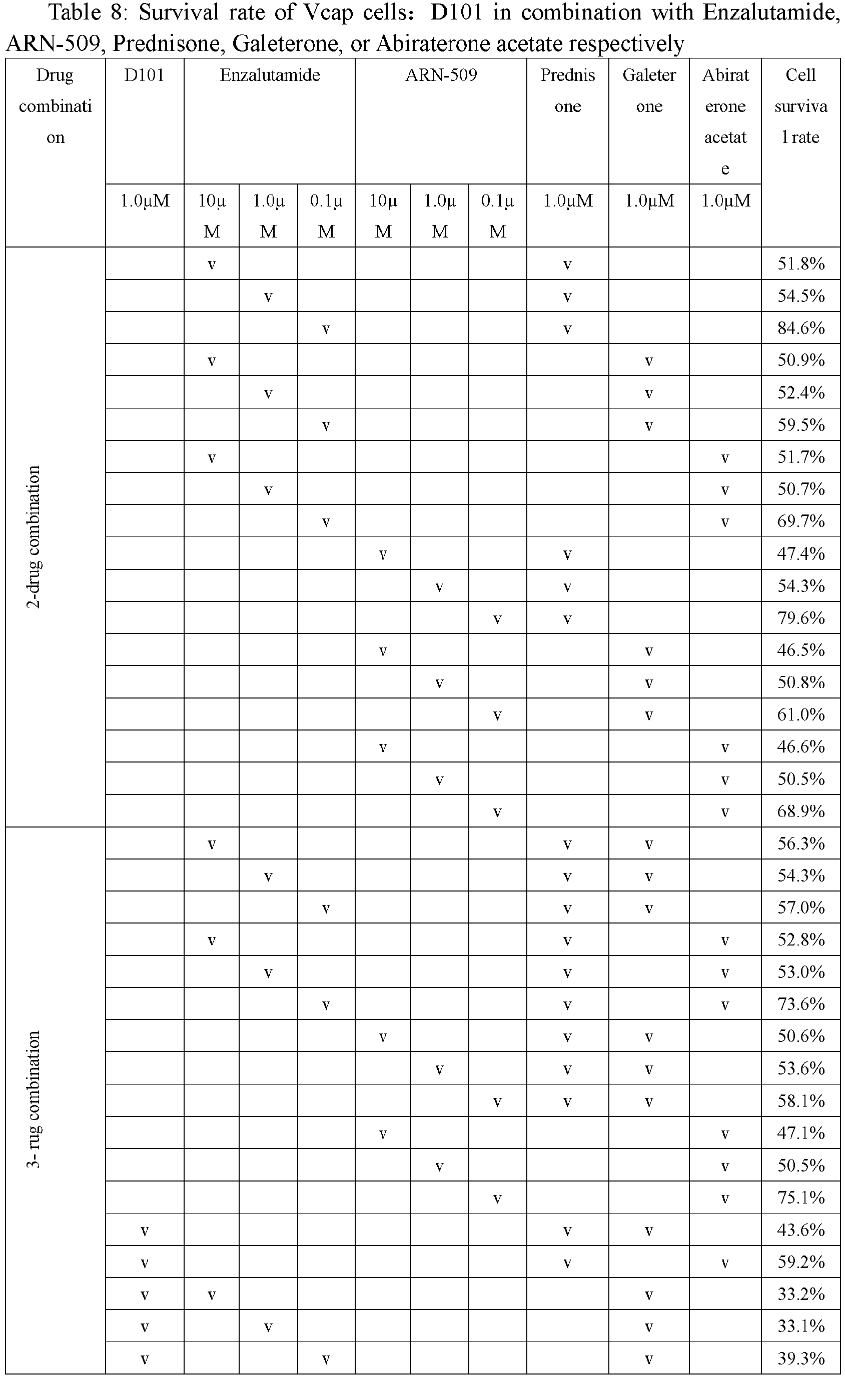

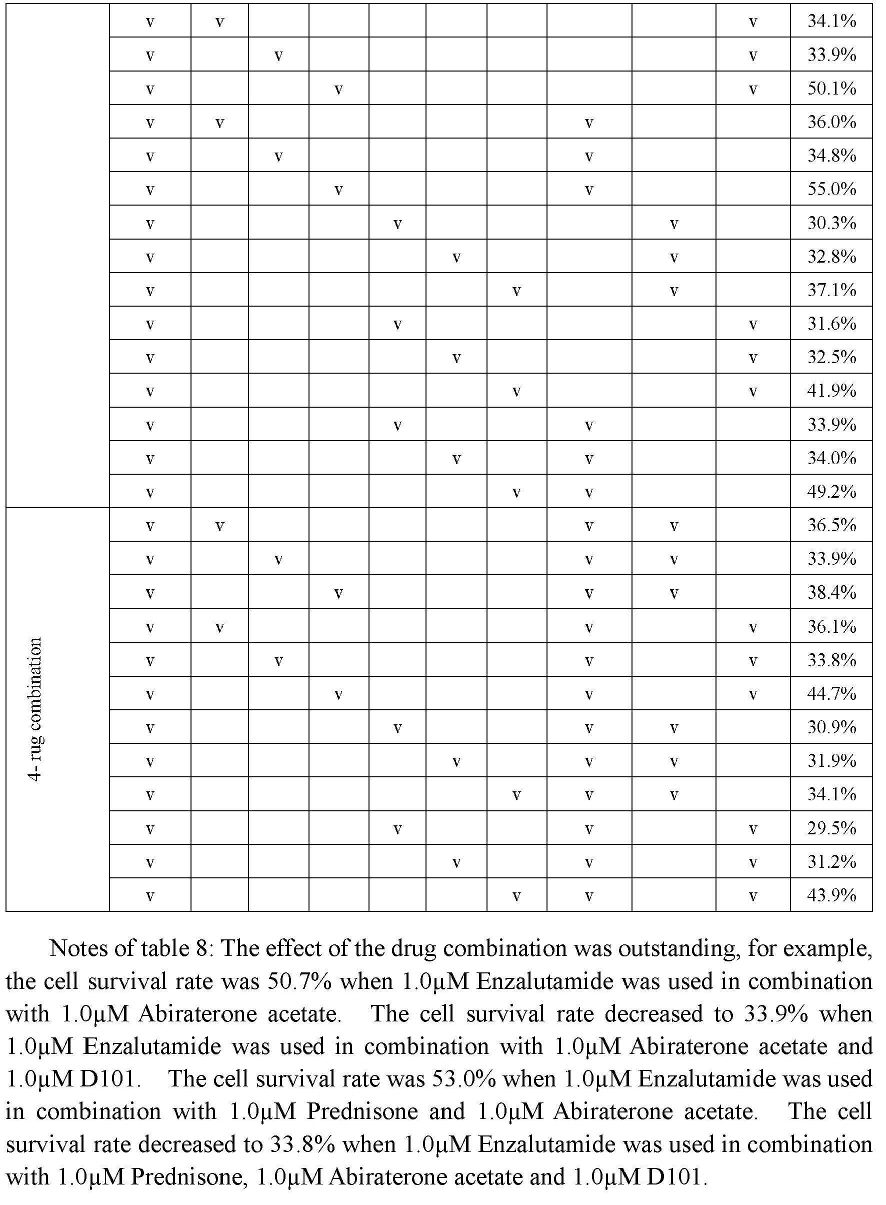

**Table 9: Survival rate of Vcap cells: Survival rate of Vcap cells: tested compound alone tested compound in combination with Enzalutamide or ARN-509**

| Tested compound | Tested compounds alone | | | In combination with 1 µM Enzalutamide | | | In combination with 1 µM ARN-509 | | |
|---|---|---|---|---|---|---|---|---|---|
| Concentration of the tested compound | 10µM | 1.0µM | 0.1µM | 10µM | 1.0µM | 0.1µM | 10µM | 1.0µM | 0.1µM |
| B001 | 69.1% | 74.8% | 83.9% | 38.4% | 42.3% | 52.1% | 40.9% | 42.6% | 50.9% |
| B002 | 75.9% | 79.8% | 85.6% | 43.1% | 44.7% | 53.9% | 44.3% | 44.7% | 52.5% |
| B003 | 80.4% | 81.3% | 83.7% | 44.2% | 45.4% | 53.5% | 42.4% | 46.5% | 54.0% |
| B004 | 88.0% | 88.4% | 94.1% | 51.1% | 50.8% | 60.4% | 48.0% | 51.2% | 59.1% |
| B005 | 83.5% | 85.0% | 91.1% | 46.5% | 48.4% | 56.5% | 46.2% | 47.6% | 53.4% |
| B006 | 85.7% | 89.7% | 91.7% | 48.3% | 50.5% | 61.2% | 45.0% | 51.7% | 63.2% |
| Enzalutamide | 58.4% | 65.7% | 85.9% | / | / | / | / | / | / |
| ARN-509 | 61.9% | 70.4% | 95.9% | / | / | / | / | / | / |

| | | | | | | | | | |
|---|---|---|---|---|---|---|---|---|---|
| Notes of table 9: The effect of the drugs combination was outstanding, for example, the cell survival rates were 65.7% and 70.4% when Enzalutamide and ARN-509 were used alone respectively. The cell survival rates were 42.3%, 44.7%, 45.4%, 50.8%, 48.4% and 50.5% when 1.0 µM Enzalutamide was used in combination with 1.0 µM B001, B002, B003, B004, B005 or B006 respectively. The cell survival rates were 42.6%, 44.7%, 46.5%, 51.2%, 47.6% and 51.7% when 1.0 µM Enzalutamide was used in combination with 1.0 µM B001 B002, B003, B004, B005 or B006 respectively. | | | | | | | | | |

**Table 10: Comparision of survival rate of Vcap cells: tested compound alone, tested compound in combination with B001 or K001**

| Tested compound | Tested compound alone | | | In combination with 1 µM B001 | | | In combination with 1µM K001 | | |
|---|---|---|---|---|---|---|---|---|---|
| Concentration of the tested compound | 10µM | 1.0µM | 0.1µM | 10µM | 1.0µ M | 0.1µ M | 10µM | 1.0µ M | 0.1µ M |
| B001 | 69.1% | 74.7% | 83.9% | / | / | / | / | / | / |
| K001 | 66.1% | 73.8% | 91.2% | / | / | / | / | / | / |
| Prednisone | 99.4% | 99.7% | 99.1% | 73.8 % | 78.7% | 87.6% | 68.0 % | 74.2% | 91.5% |
| Abiraterone | 81.9% | 92.4% | 98.5% | 66.3 % | 74.9% | 78.9% | / | / | / |
| bicalutamide | 92.0% | 94.0% | 99.2% | 67.1 % | 69.0% | 77.1% | 61.5 % | 64.1% | 78.7% |
| MK-2866 | 69.4% | 77.4% | 76.5% | 57.8 % | 65.6% | 64.8% | / | / | / |
| ARN-509 | 61.9% | 70.4% | 95.9% | 40.9 % | 42.6% | 50.9% | 41.8 % | 45.1% | 54.1% |
| Dehydroepiandrosteron e | 100.4 % | 102.4 % | 101.2 % | 85.9 % | 90.6% | 90.7% | / | / | / |
| Epiandrosterone | 90.4% | 82.0% | 87.3% | 79.7 % | 71.0% | 77.5% | / | / | / |
| Abiraterone acetate | 60.5% | 97.0% | 96.1% | 66.9 % | 70.5% | 79.8% | / | / | / |

### Effect embodiment 2 PSA inhibition rate experiment

The purpose of this experiment was to test the change of the secretion level of PSA (Prostate antigen) in the supernatant of VCap cells processed by the tested compounds in combination with Enzalutamide for 5 days.

VCap cells were processed by 0.5 µM Enzalutamide alone, and by different concentrations of 3 tested compounds in combination with 0.5 µM Enzalutamide for 5 days respectively, then the PSA level of each treatment group was tested by electrochemiluminescence immunoassay.

### Experimental materials and methods

### 1. Cell line

| Type of tumor | Name of cell | Medium | Processing time (days) |
|---|---|---|---|
| prostate cancer | VCap | DMEM+10%FBS | 5 |

The cell culture conditions were: 37°C, 5% CO₂ and 95% humidity.

### 2. Reagents

1) DMEM medium (Thermo scientific, product number: SH30243.01)
2) FBS (Fetal Bovine Serum) (Gibco, product number: 10099-141)
3) 0.25% trypsin-EDTA (Gibco, product number: 25200-072)
4) DMSO (Sigma, product number: D2650)
5) Prostate specific antigen reagent (Roche, product number: 04641655190) (provided by Taicang First People's Hospital)

### 3. Equipments

1) Carbon dioxide incubator: SANYO Electric Co., Ltd. (Japan). (Equipment ID: TAINC0490)
2) Microscope: Chongguang XDS-1B, Chongqing Guangdian Corp. (Chongqing, P.R.China). (Equipment ID: TAMIC0130)
3) Refrigerator: Haier Z16TXZ (China). (Equipment ID: TAREF0490)
4) Electronic Balance: Mettlertoledo AL104. (Shanghai, China). (Equipment ID: TBBAL0560)
5) Automatic Electrochemical Immunoassay Analyzer: Roche Cobas e601 (Taicang First People's Hospital)

### 4. Secretion inhibition rate of the tested compounds on VCap cell PSA

### Cell inoculation

Cells were collected in exponential growth phase for viable cell count. The cell suspension was adjusted to 4.17×10e⁴/ml with the medium mentioned above. 120µl of cell suspension was added to each well of a 96-well cell culture plateat a final concentration of cells was 5000 cells/well. The cells were incubated overnight in a 37°C, 5% CO₂ incubator.

### Dosing treatment

10mM stocking solution was prepared by dissolving each tested compound in DMSO. A series of 4× serial gradient dilutions were prepared with the stocking solution and DMSO, followed by being diluted with medium to be 10-fold dilutions respectively, and a 10-fold solution of Enzalutamide was prepared meanwhile. Enzalutamide and the equivalent volume of corresponding solution of the tested compound were added to each well for each cell line respectively, and a duplicate well was set for each drug concentration. The final concentrations of Enzalutamide and the tested compound used in the test are shown in Table 11. The final concentration of DMSO per well was 0.2%. The cells were incubated for 5 days in a 37°C, 5% CO₂ incubator.

### Detection

After 5 days' drug treatment, the cell supernatant of each well was collected, and centrifuged at 2000 r/min for 5 minutes, then transferred to a clean EP tube for PSA detection.

### 5. Data analysis

Calculation formula of PSA inhibition rate: (1-(Vₛₐₘₚₗₑ/V_{DMSO})) × 100%. Wherein, Vₛₐₘₚₗₑ is the PSA reading of the drug treatment group, and V_{DMSO} is the average value of PSA of the solvent control group.

**Table 11 PSA inhibition rate(%): 0.5 µM Enzalutamide used alone, and in combination with different concentrations of the tested compound**

| | | | | | | | | |
|---|---|---|---|---|---|---|---|---|
| Concentration of the tested compound (µM) | 100 | 25 | 6.25 | 1.56 | 0.39 | 0.10 | 0.02 | 0.01 |
| Enzalutamide (0.5µM) + K001 | 37.3 % | 50% | 55.2 % | 52.7 % | 52.7 % | 55% | 53% | 50% |
| Enzalutamide (0.5µM) + B001 | 65.4 % | 63% | 63.2 % | 63.9 % | 59.7 % | 50% | 46.7 % | 43.6 % |
| Enzalutamide (0.5µM) + D108 | 69.2 % | 60.3 % | 61.6 % | 57.9 % | 57.9 % | 57.2 % | 56.8 % | 56.1 % |
| PSA inhibition rate was 30.7% when Enzalutamide (0.5µM) was used alone | | | | | | | | |

| | | | | | | | | |
|---|---|---|---|---|---|---|---|---|
| Notes of Table 11: The PSA inhibition rate was 30.7% when 0.5µM Enzalutamide was used alone. The PSA inhibition rates were 55%, 50% and 57.2% when 0.5µM Enzalutamide was used in combination with 0.1µM K001, B001 and D 108 respectively. It can be seen that the effect of drug combination was significantly enhanced compared with Enzalutamide used alone. | | | | | | | | |

Although the specific embodiments of the present invention are described above, it will be understood by people skilled in the art that these are just examples. Therefore, the protection scope of the present invention is defined by the claims attached.

## Claims

1. A pharmaceutical composition, comprising: i) a benzoheterocyclic compound, a pharmaceutically acceptable salt thereof, or a stereoisomer thereof, and ii) one or more androgen receptor pathway modulator;
wherein the combination of the benzoheterocyclic compound and the androgen receptor pathway modulator is that: B001 and Enzalutamide, K001 and Enzalutamide, D107 and Enzalutamide, B001 and ARN-509, K001 and ARN-509, D107 and ARN-509, B001 and Galeterone, K001 and Galeterone, D107 and Galeterone, B001 and ODM-201, K001 and ODM-201, D108 and ODM-201, F001 and ODM-201, B002 and Enzalutamide, B003 and Enzalutamide, B004 and Enzalutamide, B005 and Enzalutamide, B006 and Enzalutamide, B002 and ARN-509, B003 and ARN-509, B004 and ARN-509, B005 and ARN-509, B006 and ARN-509, B001 and Enzalutamide and Galeterone, B001 and Enzalutamide and Abiraterone acetate, B001 and ARN-509 and Abiraterone acetate, B001 and ARN-509 and Galeterone, K001 and Enzalutamide and Galeterone, K001 and Enzalutamide and Abiraterone acetate, K001 and ARN-509 and Abiraterone acetate, K001 and ARN-509 and Galeterone;
wherein the structures of B001, B002, B003, B004, B005, B006, F001, K001, D107 and D108 are as follows: 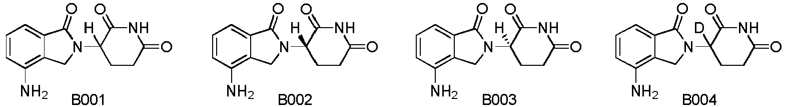 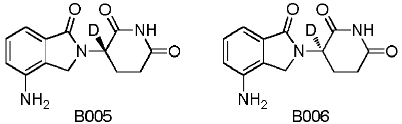 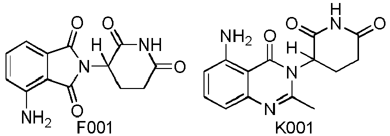 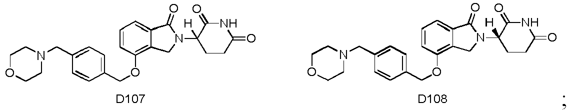 and
wherein
the structure of ARN-509 is 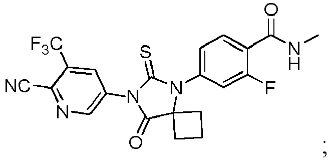
the structure of ODM-201 is

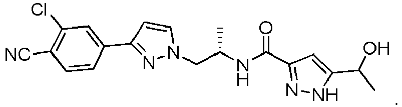

2. A pharmaceutical composition comprising: i) a benzoheterocyclic compound, a pharmaceutically acceptable salt thereof, or a stereoisomer thereof, and ii) one or more androgen receptor pathway modulator and iii) a hormone compound;
wherein the benzoheterocyclic compound is B001, K001 or D101; and
wherein the combination of the androgen receptor pathway modulator and the hormone compound is prednisone and Abiraterone acetate, Enzalutamide and prednisone, ARN-509 and prednisone, Enzalutamide and Galeterone and prednisone, Enzalutamide and Abiraterone acetate and prednisone, ARN-509 and Galeterone and prednisone, ARN-509 and Abiraterone acetate and prednisone;
wherein the structures of B001, K001 and D101 are as follows: 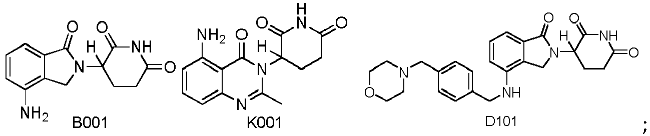 and
wherein
the structure of ARN-509 is

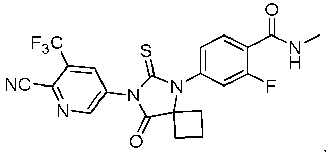

3. The pharmaceutical composition according to claim 1 for use in the prevention and/or treatment of prostate cancer.

4. The pharmaceutical composition for use according to claim 3, wherein the benzoheterocyclic compound and the androgen receptor pathway modulator are administered simultaneously, or in a sequential order, or separately.

5. The pharmaceutical composition according to claim 2 for use in the prevention and/or treatment of prostate cancer.

6. The pharmaceutical composition for use according to claim 5, wherein the benzoheterocyclic compound, the androgen receptor pathway modulator and the hormone compound are administered simultaneously, or in a sequential order, or separately.

## Patentansprüche

1. Pharmazeutische Zusammensetzung, die aufweist: i) eine benzoheterozyklische Verbindung, ein pharmazeutisch akzeptables Salz davon, oder ein Stereoisomer davon, und ii) einen oder mehrere Androgenrezeptorwegmodulatoren;
wobei die Kombination aus der benzoheterozyklischen Verbindung und dem Androgenrezeptorwegmodulator wie folgt ist: B001 und Enzalutamid, K001 und Enzalutamid, D107 und Enzalutamid, B001 und ARN-509, K001 und ARN-509, D107 und ARN-509, B001 und Galeteron, K001 und Galeteron, D107 und Galeteron, B001 und ODM-201, K001 und ODM-201, D108 und ODM-201, F001 und ODM-201, B002 und Enzalutamid, B003 und Enzalutamid, B004 und Enzalutamid, B005 und Enzalutamid, B006 und Enzalutamid, B002 und ARN-509, B003 und ARN-509, B004 und ARN-509, B005 und ARN-509, B006 und ARN-509, B001 und Enzalutamid und Galeteron, B001 und Enzalutamid und Abirateronacetat, B001 und ARN-509 und Abirateronacetat, B001 und ARN-509 und Galeteron, K001 und Enzalutamid und Galeteron, K001 und Enzalutamid und Abirateronacetat, K001 und ARN-509 und Abirateronacetat, K001 und ARN-509 und Galeteron;
wobei die Strukturen von B001, B002, B003, B004, B005, B006, F001, K001, D107 und D108 wie folgt aussehen: 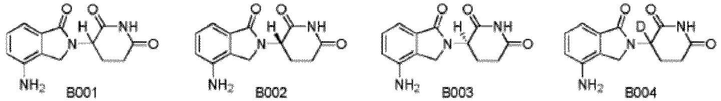 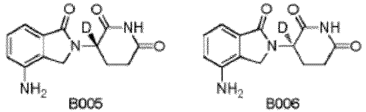 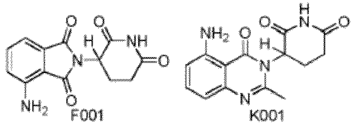 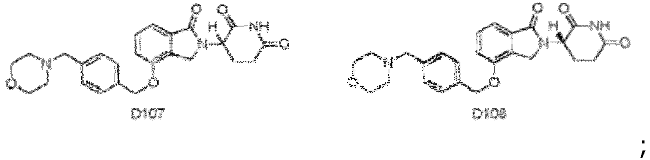 und
wobei
die Struktur von ARN-509 ist 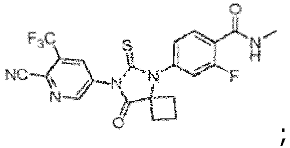
die Struktur von ODM-201 ist

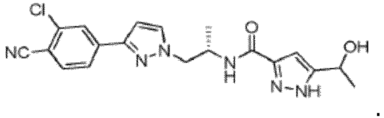

2. Pharmazeutische Zusammensetzung, die aufweist: i) eine benzoheterozyklische Verbindung, ein pharmazeutisch akzeptables Salz davon, oder ein Stereoisomer davon, und ii) einen oder mehrere Androgenrezeptorwegmodulatoren, und iii) ein Hormonpräparat;
wobei die benzoheterozyklische Verbindung B001, K001 oder D101 ist; und
wobei die Kombination aus dem Androgenrezeptorwegmodulator und dem Hormonpräparat Prednison und Abirateronacetat, Enzalutamid und Prednison, ARN-509 und Prednison, Enzalutamid und Galeteron und Prednison, Enzalutamid und Abirateronacetat und Prednison, ARN-509 und Galeteron und Prednison, ARN-509 und Abirateronacetat und Prednison ist;
wobei die Strukturen von B001, K001 und D101 wie folgt aussehen: 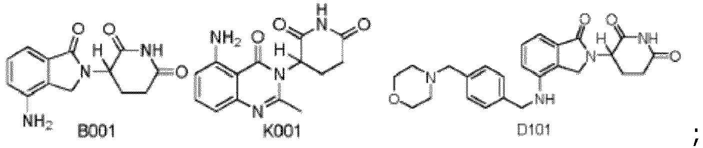 und
wobei
die Struktur von ARN-509 ist

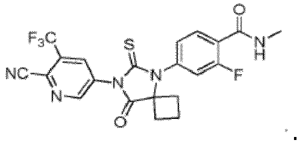

3. Pharmazeutische Zusammensetzung nach Anspruch 1 zur Verwendung bei der Prävention und/oder Behandlung von Prostatakrebs.

4. Pharmazeutische Zusammensetzung zur Verwendung nach Anspruch 3, wobei die benzoheterozyklische Verbindung und der Androgenrezeptorwegmodulator gleichzeitig, oder nacheinander, oder separat verabreicht werden.

5. Pharmazeutische Zusammensetzung nach Anspruch 2 zur Verwendung bei der Prävention und/oder Behandlung von Prostatakrebs.

6. Pharmazeutische Zusammensetzung zur Verwendung nach Anspruch 5, wobei die benzoheterozyklische Verbindung, der Androgenrezeptorwegmodulator, und das Hormonpräparat gleichzeitig, oder nacheinander, oder separat verabreicht werden.

## Revendications

1. Composition pharmaceutique, comprenant : i) un composé benzohétérocyclique, un sel pharmaceutiquement acceptable de celui-ci, ou un stéréoisomère de celui-ci, et ii) un ou plusieurs modulateurs de voie du récepteur des androgènes ;
dans laquelle la combinaison du composé benzohétérocyclique et du modulateur de voie du récepteur des androgènes est celle-ci : B001 et enzalutamide, K001 et enzalutamide, D107 et enzalutamide, B001 et ARN-509, K001 et ARN-509, D107 et ARN-509, B001 et galétérone, K001 et galétérone, D107 et galétérone, B001 et ODM-201, K001 et ODM-201, D108 et ODM-201, F001 et ODM-201, B002 et enzalutamide, B003 et enzalutamide, B004 et enzalutamide, B005 et enzalutamide, B006 et enzalutamide, B002 et ARN-509, B003 et ARN-509, B004 et ARN-509, B005 et ARN-509, B006 et ARN-509, B001 et enzalutamide et galétérone, B001 et enzalutamide et acétate d'abiratérone, B001 et ARN-509 et acétate d'abiratérone, B001 et ARN-509 et galétérone, K001 et enzalutamide et galétérone, K001 et enzalutamide et acétate d'abiratérone, K001 et ARN-509 et acétate d'abiratérone, K001 et ARN-509 et galétérone ;
dans laquelle les structures de B001, B002, B003, B004, B005, B006, F001, K001, D107 et D108 sont les suivantes : 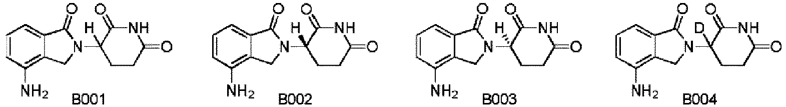 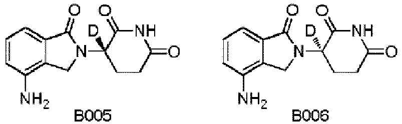 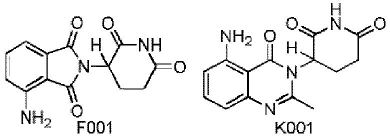 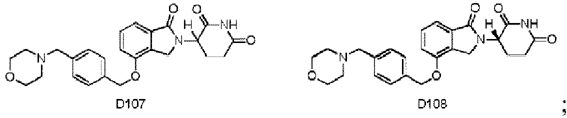 et
dans laquelle
la structure d'ARN-509 est 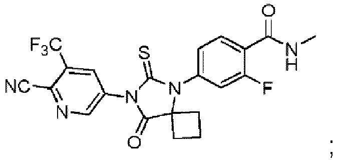
la structure d'ODM-201 est

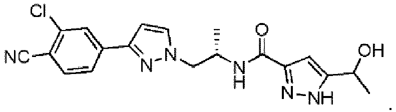

2. Composition pharmaceutique comprenant : i) un composé benzohétérocyclique, un sel pharmaceutiquement acceptable de celui-ci, ou un stéréoisomère de celui-ci, et ii) un ou plusieurs modulateurs de voie du récepteur des androgènes et iii) un composé hormonal ;
dans laquelle le composé benzohétérocyclique est B001, K001 ou D101 ; et
dans laquelle la combinaison du modulateur de voie de récepteur des androgènes et du composé hormonal est prednisone et acétate d'abiratérone, enzalutamide et prednisone, ARN-509 et prednisone, enzalutamide et galétérone et prednisone, enzalutamide et acétate d'abiratérone et prednisone, ARN-509 et galétérone et prednisone, ARN-509 et acétate d'abiratérone et prednisone ;
dans laquelle les structures de B001, K001 et D101 sont les suivantes : 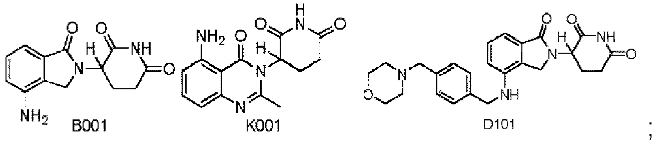 et
dans laquelle
la structure d'ARN-509 est

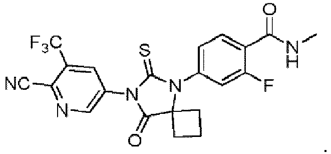

3. Composition pharmaceutique selon la revendication 1 pour une utilisation dans la prévention et/ou le traitement du cancer de la prostate.

4. Composition pharmaceutique pour une utilisation selon la revendication 3, dans laquelle le composé benzohétérocyclique et le modulateur de voie du récepteur des androgènes sont administrés simultanément, ou dans un ordre séquentiel, ou séparément.

5. Composition pharmaceutique selon la revendication 2 pour une utilisation dans la prévention et/ou le traitement du cancer de la prostate.

6. Composition pharmaceutique pour une utilisation selon la revendication 5, dans laquelle le composé benzohétérocyclique, le modulateur de voie du récepteur des androgènes et le composé hormonal sont administrés simultanément, ou dans un ordre séquentiel, ou séparément.
